# EUROPEAN PATENT APPLICATION

(11) **EP 0 761 215 A1**
(43) Date of publication of application: **12.03.1997**
(21) Application number: 95923537.5
(22) Date of filing: 28.06.1995
(51) Int. Cl.: A61K 31/275, A61K 31/34, A61K 31/38, A61K 31/42, A61K 31/425, A61K 31/44, A61K 31/445, A61K 31/495, A61K 31/505, A61K 31/535, A61K 31/55

(54) **ANTIBACTERIAL AGENT**

(30) Priority: 29.06.1994 JP 147466/94; 07.04.1995 JP 82615/95
(71) Applicant: DAIICHI PHARMACEUTICAL CO., LTD., Chuo-ku, Tokyo 103 (JP)
(72) Inventor: KATO, Shozo Tokuyama Corporation, Tokuyama-shi Yamaguchi 745 (JP); ITAHANA, Hidenobu Daiichi Pharmaceutical Co., Ltd., Tokyo 134doga (JP); ISHIZAKI, Masahiko +di, - (JP); FURUKI, Makiko Daiichi Pharmaceutical Co., Ltd., Tokyo 134dogawa- (JP); YAMAGUCHI, Masao Tokuyama Corporation, Tokuyama-shi Yamaguchi 745 (JP); OHMURO, Fumio Daiichi Pharmaceutical Co., Ltd., Tokyo 134 (JP); NAGATA, Seiji Tokuyama Corporation, Tokuyama-shi Yamaguchi 745 (JP); OHARA, Eiji Daiichi Pharmaceutical Co., Ltd., Tokyo 104 (JP); KITAJIMA, Toshio Tokuyama Corporation, Tokuyama-shi Yamaguchi 745 (JP)
(74) Representative: Wächtershäuser, Günter, Prof. Dr.
(86) International application number: JP9501289
(87) International publication number: WO9600570

(57) **Abstract**

The present invention provides an antibacterial agent containing, as an active component, a ketonitrile derivative represented by the following formula (1) or a salt thereof: 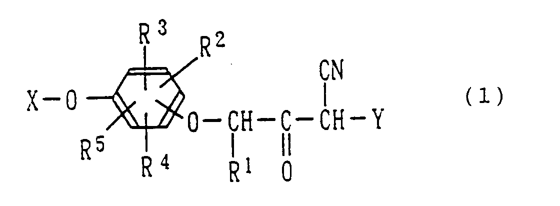 (wherein R¹ is a hydrogen atom or lower alkyl, each of R², R³, R⁴, and R⁵, which are identical to or different from one another, is a hydrogen atom, a halogen atom, nitro, lower alkyl, lower alkoxyl, or substituted or unsubstituted phenoxy, X is a substituted or unsubstituted aromatic hydrocarbon group or a substituted or unsubstituted heterocyclic group, and Y is a substituted or unsubstituted aromatic hydrocarbon group, a substituted or unsubstituted heterocyclic group, a carbonyl group substituted by a substituted or unsubstituted aromatic hydrocarbon group or by a substituted or unsubstituted heterocyclic group, or a N,N-di-substituted carbamoyl group). This compound possesses excellent antibacterial activity against both gram-positive and gram-negative bacteria.

## Description

### Technical Field:

The present invention relates to antibacterial agents which are used for preventing and treating infectious diseases of humans and animals such as cattle. According to the invention, antibacterial agents which combat both gram-negative and gram-positive bacteria are provided.

### Background Art:

In infectious diseases of humans and animals such as cattle, birds, and fish caused by causal bacteria such as *Staphylococcus aureus*, hemolytic *Streptococci*, *Bacillus anthracis* and *Clostridium tetani*, antibiotics or synthetic antibacterial agents including penicillins, tetracyclines, macrolides, cephalosporins, and cephems have conventionally been used.

Prolonged terms of use or a large amount of use of these conventional antibiotics or synthetic antibacterial agents, however, causes drug resistance to the aforementioned causal bacteria. Especially, methicillin-resistant *Staphylococcus aureus* (MRSA), which causes a high death rate due to its strong toxicity, has become a big social problem, for it exhibits resistance to multiple drugs and makes therapy difficult.

The present inventors conducted extensive research to solve the above problem, and found that certain ketonitrile derivatives which have heretofore been known to possess herbicide activities (see Japanese Patent Application Laid-open (*kokai*) Nos. 5-148,213, 6-179,646 and 7-109,249), and even some ketonitrile derivatives which have heretofore been acknowledged not to possess herbicide activities, exhibit antibacterial activities, and in particular, high antibacterial activities against gram-positive bacteria such as *Staphylococci* and *Streptococci*, as well as gram-negative bacteria, leading to completion of the invention.

### Disclosure of the Invention

The present invention is directed to an antibacterial agent containing, as an active component, a ketonitrile derivative represented by the following formula (1) or a salt thereof: 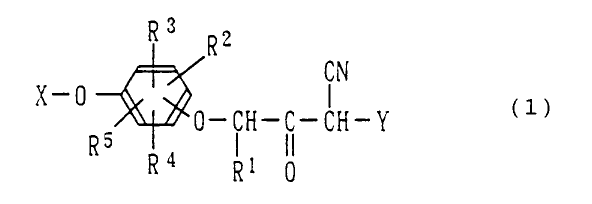 (wherein R¹ is a hydrogen atom or lower alkyl, each of R², R³, R⁴, and R⁵, which are identical to or different from one another, is a hydrogen atom, a halogen atom, nitro, lower alkyl, lower alkoxyl, or substituted or unsubstituted phenoxy, X is a substituted or unsubstituted aromatic hydrocarbon group or a substituted or unsubstituted heterocyclic group, Y is a substituted or unsubstituted aromatic hydrocarbon group, a substituted or unsubstituted heterocyclic group, a carbonyl group substituted by a substituted or unsubstituted aromatic hydrocarbon group or by a substituted or unsubstituted heterocyclic group, or a N,N-di-substituted carbamoyl group).

### Best Mode for Carrying Out the Invention

In formula (1), examples of the lower alkyl groups represented by R¹ include methyl, ethyl, propyl, and isopropyl. Preferably, R¹ is a hydrogen atom or methyl.

In formula (1), examples of each one of R² through R⁵ include a hydrogen atom; halogen atoms such as chlorine, bromine, fluorine, and iodine; nitro; lower alkyl groups such as methyl, ethyl, propyl, and butyl; and lower alkoxyl groups such as methoxy, ethoxy, and propoxy; phenoxy groups such as 2-chloro-4-trifluoromethylphenoxy.

In formula (1), examples of the unsubstituted aromatic hydrocarbon groups represented by X include phenyl and naphthyl. Examples of the unsubstituted heterocyclic groups also represented by X include groups constituted by 5-membered or 6-membered rings containing one or more heterologous atoms of oxygen, sulfur, or nitrogen; or groups which are formed by condensation of these groups and a benzene ring, for example, aromatic heterocyclic groups such as pyridyl, quinolyl, isoquinolyl, pyrimidinyl, oxazolyl, benzoxazolyl, furyl, benzofuryl, thiazolyl, benzothiazolyl, thienyl, and benzothienyl; and non-aromatic heterocyclic groups such as piperidinyl, pyrrolidinyl, morpholinyl, tetrahydrofuryl, and tetrahydrothienyl. Especially, aromatic heterocyclic groups are preferred.

When group X is a substituted aromatic hydrocarbon group or a substituted heterocyclic group, examples of the substituent include halogen atoms such as chlorine, fluorine, bromine, and iodine; haloalkyl groups such as trifluoromethyl; nitro, cyano, alkyl, alkylthio, and alkoxycarbonyl. The number of substituents may be one or more. Especially, it is preferred that the substituent or substituents be one member or homologous or heterologous two members selected from the group consisting of halogen atoms and haloalkyl, in view that the resulting compounds exhibit enhanced antibacterial activities.

In formula (1), examples of the unsubstituted aromatic hydrocarbon groups represented by Y include phenyl and naphthyl. Examples of the unsubstituted heterocyclic groups, which are also represented by Y, include groups constituted by 5-membered or 6-membered rings each containing one or more heterologous atoms of oxygen, sulfur, or nitrogen; or groups which are formed by condensation of these groups and a benzene ring, for example, the heterocyclic groups mentioned above with respect to group X. Especially, aromatic heterocyclic groups are preferred.

When group Y is a substituted aromatic hydrocarbon group, a substituted heterocyclic group, or a carbonyl group substituted by one of substituted aromatic hydrocarbon groups or substituted heterocyclic groups, examples of the substituent include halogen atoms such as chlorine, fluorine, bromine, and iodine; haloalkyl groups such as trifluoromethyl; alkyl, alkoxyl, alkylthio, and alkoxycarbonyl.

Examples of the N,N-di-substituted carbamoyl group include substituted or unsubstituted alkyl, substituted or unsubstituted alkenyl, substituted or unsubstituted alkoxyl, substituted or unsubstituted aromatic groups, or carbamoyl groups which are substituted by substituted or unsubstituted heterocyclic groups. Alternatively, the two substituents which are bound to the carbamoyl group may join to form a ring which may contain a heterologous atom (examples of such a ring include pyrrolidine, piperidine, and morpholine). Also, 1-azacycloalkylcarbonyl groups or the like which may be condensed with a benzene ring or which may be substituted by an alkyl group can be used.

The compounds of formula (1) are divided into the following three groups represented by formulas (2), (3), and (4). In the present invention, compounds belonging to any of these compound groups can be used. 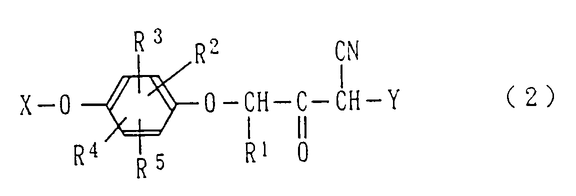 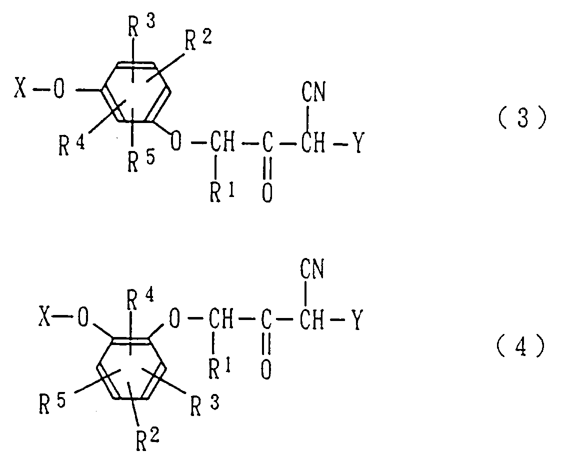 (wherein R¹, R², R³, R⁴, R⁵, X, and Y have the same meanings as defined above).

In formula (2), group X is preferably substituted or unsubstituted phenyl, substituted or unsubstituted pyridyl, substituted or unsubstituted benzoxazolyl, or substituted or unsubstituted benzothiazolyl. Particularly, phenyl which may be substituted by a halogen atom, haloalkyl or cyano; pyridyl which may be substituted by a halogen atom, haloalkyl or cyano; benzoxazolyl which may be substituted by a halogen atom, haloalkyl or cyano; or benzothiazolyl which may be substituted by a halogen atom, haloalkyl or cyano are preferred.

In formula (2), Y is preferably substituted or unsubstituted phenyl, substituted or unsubstituted pyrimidinyl, substituted or unsubstituted thienyl, substituted or unsubstituted naphthyl, or N,N-di-substituted carbamoyl. Particularly, phenyl which may be substituted by a halogen atom, haloalkyl, alkyl, or alkoxyl; pyrimidinyl which may be substituted by a halogen atom, haloalkyl, alkyl, or alkoxyl; thienyl which may be substituted by a halogen atom, haloalkyl, alkyl, or alkoxyl; or naphthyl which may be substituted by a halogen atom, haloalkyl, alkyl, or alkoxyl; N,N-di-substituted carbamoyl substituted by lower alkyl, aryl, or aralkyl; or 1-azacycloalkylcarbonyl which may be condensed with a benzene ring or which may be substituted by an alkyl group are preferred.

In formula (2), X and Y are preferably substituted or unsubstituted aromatic heterocyclic groups, of which preferable examples of X include substituted or unsubstituted phenyl, substituted or unsubstituted pyridyl, substituted or unsubstituted benzoxazolyl, or substituted or unsubstituted benzothiazolyl, and preferable examples of Y include substituted or unsubstituted phenyl, substituted or unsubstituted pyrimidinyl, substituted or unsubstituted thienyl, substituted or unsubstituted naphthyl, or N,N-di-substituted carbamoyl. More preferably, X is phenyl which may be substituted by a halogen atom, haloalkyl, or cyano; pyridyl which may be substituted by a halogen atom, haloalkyl, or cyano; benzoxazolyl which may be substituted by a halogen atom, haloalkyl, or cyano; or benzothiazolyl which may be substituted by a halogen atom, haloalkyl, or cyano; and Y is phenyl which may be substituted by a halogen atom, haloalkyl, alkyl, or alkoxyl; pyrimidyl which may be substituted by a halogen atom, haloalkyl, alkyl, or alkoxyl; thienyl which may be substituted by a halogen atom, haloalkyl, alkyl, or alkoxyl; naphthyl which may be substituted by a halogen atom, haloalkyl, alkyl, or alkoxyl; N,N-di-substituted carbamoyl substituted by lower alkyl, aryl, or aralkyl; or 1-azacycloalkylcarbonyl which may be condensed with a benzene ring or which may be substituted by an alkyl group.

In formula (2), more preferable combinations of X and Y are as follows: X is substituted or unsubstituted pyridyl and Y is substituted or unsubstituted phenyl, substituted or unsubstituted pyrimidinyl, or N,N-di-substituted carbamoyl; X is substituted or unsubstituted phenyl and Y is substituted or unsubstituted pyrimidinyl, or N,N-di-substituted carbamoyl; X is substituted or unsubstituted benzoxazolyl or substituted or unsubstituted benzothiazolyl and Y is substituted or unsubstituted phenyl.

Among the ketonitrile derivatives represented by formula (2), 4-[4-(3-chloro-5-trifluoromethyl-2-pyridyloxy)-phenoxy]-2-(3,4-dichlorophenyl)-3-oxopentanonitrile (Compound No. 4) is especially preferred because of its prominent antibacterial activities.

In formula (3), X is preferably a substituted or unsubstituted aromatic hydrocarbon group or a substituted or unsubstituted heterocyclic group. Particularly preferably, X is substituted or unsubstituted phenyl, or substituted or unsubstituted pyridyl. Specifically, phenyl that has been substituted by a halogen atom or haloalkyl and pyridyl that has been substituted by a halogen atom or haloalkyl are preferred.

In formula (3), Y is preferably substituted or unsubstituted phenyl, substituted or unsubstituted naphthyl, substituted or unsubstituted pyridyl, or N,N-di-substituted carbamoyl. More preferably, phenyl which may be substituted by a halogen atom, haloalkyl, or alkoxyl and N,N-di-substituted carbamoyl are preferred. Particularly, phenyl which is substituted by a halogen atom or haloalkyl is preferred.

In formula (3), preferable combinations of X and Y are as follows: X is substituted phenyl or substituted pyridyl and Y is substituted or unsubstituted phenyl, substituted or unsubstituted pyridyl, or N,N-di-substituted carbamoyl. Especially, combinations in which X is phenyl substituted by a halogen atom or a haloalkyl group or pyridyl substituted by a halogen atom or a haloalkyl group; and Y is phenyl which may be substituted by a halogen atom, a haloalkyl group, or an alkoxyl group are preferred.

Among the ketonitrile derivatives represented by formula (3), the following compounds are preferred due to their prominent antibacterial activities:
2-(3-bromophenyl)-4-[2-chloro-5-(2-chloro-4-trifluoromethylphenoxy)phenoxy]-3-oxopentanonitrile,
4-[3-(2-chloro-4-trifluoromethylphenoxy)phenoxy]-2-(3,4-dichlorophenyl)-3-oxopentanonitrile, and
4-[3-(2-chloro-4-trifluoromethylphenoxy)-5-methylphenoxy]-2-(3,4-dichlorophenyl)-3-oxopentanonitrile.

In formula (4), X is preferably a substituted or unsubstituted aromatic hydrocarbon group or a substituted or unsubstituted heterocyclic group. Particularly preferably, X is substituted or unsubstituted phenyl or substituted or unsubstituted pyridyl. Especially, phenyl substituted by a halogen atom or haloalkyl and pyridyl substituted by a halogen atom or haloalkyl are preferred. Specifically, phenyl which may be substituted by a halogen atom or a haloalkyl group is preferred.

In formula (4), Y is preferably substituted or unsubstituted phenyl, substituted or unsubstituted naphthyl, or substituted or unsubstituted pyridyl. More preferably, phenyl which may be substituted by a halogen atom, haloalkyl, or alkoxyl is preferred. Particularly, phenyl which has been substituted by a halogen atom or haloalkyl is preferred.

In formula (4), preferable combinations of X and Y are as follows: X is substituted phenyl or substituted pyridyl and Y is substituted or unsubstituted phenyl, or substituted or unsubstituted pyridyl. Especially, combinations in which X is phenyl that has been substituted by a halogen atom or a haloalkyl group; or pyridyl that has been substituted by a halogen atom or a haloalkyl group; and Y is unsubstituted phenyl or phenyl that has been substituted by a halogen atom haloalkyl, or alkoxyl are preferred.

Among the ketonitrile derivatives represented by formula (4), the following compounds are preferred due to their prominent antibacterial activities: 4-[2-(2-chloro-4-trifluoromethylphenoxy)-4-methylphenoxy]-2-(3,4-dichlorophenyl)-3-oxopentanonitrile, 4-[2-(2-chloro-4-trifluoromethylphenoxy)-5-methylphenoxy]-2-(3,4-dichlorophenyl)-3-oxopentanonitrile, 4-[2-(2-chloro-4-trifluoromethylphenoxy)-4-methylphenoxy]-2-(3-bromophenyl)-3-oxopentanonitrile, and 4-[2-(2-chloro-4-trifluoromethylphenoxy)-5-methylphenoxy]-2-(3-bromophenyl)-3-oxopentanonitrile.

The ketonitrile derivatives of formula (1) which are used in the present invention may be in the form of salts. The salts include, for example, inorganic salts formed with inorganic acids such as hydrochloric acid, sulfuric acid, nitric acid, hydrobromic acid, or phosphoric acid; organic salts formed with organic acids such as acetic acid, fumaric acid, maleic acid, lactic acid, tartaric acid, citric acid, malic acid, oxalic acid, methanesulfonic acid, benzenesulfonic acid, and p-toluenesulfonic acid; salts formed with acidic amino acids such as aspartic acid and glutamic acid; metal salts formed with metals such as sodium, potassium, calcium, magnesium, zinc, and silver; salts formed with organic bases such as dimethylamine, triethylamine, diethanolamine, and benzylamine; and salts formed with basic amino acids such as lysine and alginine. In the present invention, solvates of the ketonitrile derivatives (1) typified by hydrates thereof can also be used.

The ketonitrile compounds (1) which are used in the present invention may be prepared, for example, according to any one of the following methods (a) to (e):
(a): A compound represented by formula (5) and a compound represented formula (6) are reacted in the presence or absence of a solvent to obtain a ketonitrile derivative 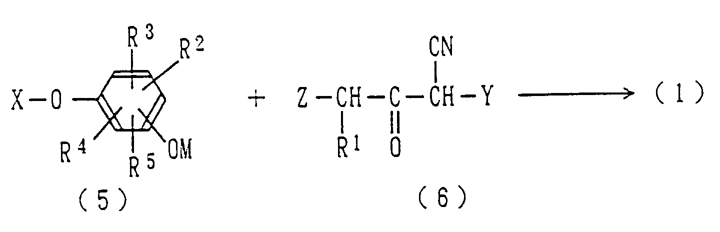 (wherein M is a hydrogen atom or alkali metal, Z is a halogen atom, and R¹, R², R³, R⁴, R⁵, X, and Y have the same meanings as defined above).
(b): A compound represented by formula (7) and a compound represented formula (8) are reacted in the presence or absence of a solvent to obtain a ketonitrile derivative 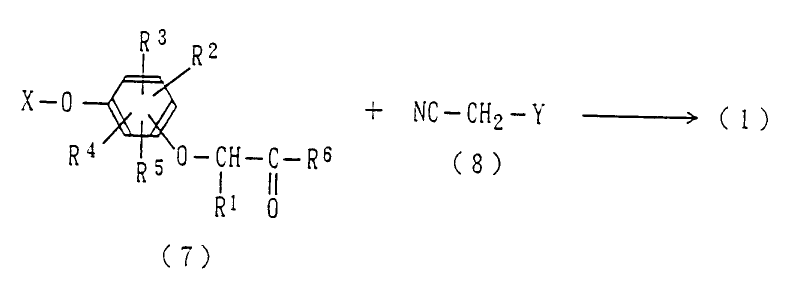 (wherein R⁶ is an alkoxyl group or a halogen atom, and R¹, R², R³, R⁴, R⁵, X, and Y have the same meanings as defined above).
(c): A compound represented by formula (9) and a compound represented formula (10) are reacted in the presence or absence of a solvent to obtain a ketonitrile derivative (1). 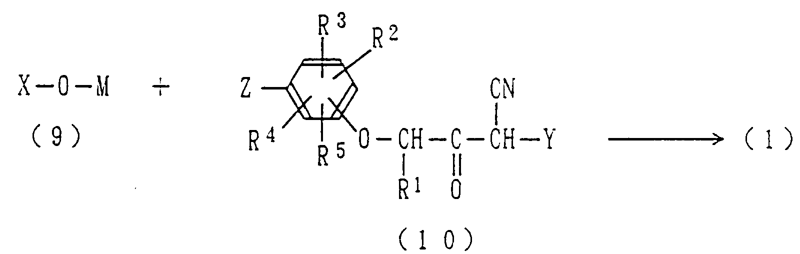 (wherein R¹, R², R³, R⁴, R⁵, M, X, Y and Z have the same meanings as defined above).
(d): A compound represented by formula (11) and a compound represented formula (12) are reacted in the presence or absence of a solvent to obtain a ketonitrile derivative (1). 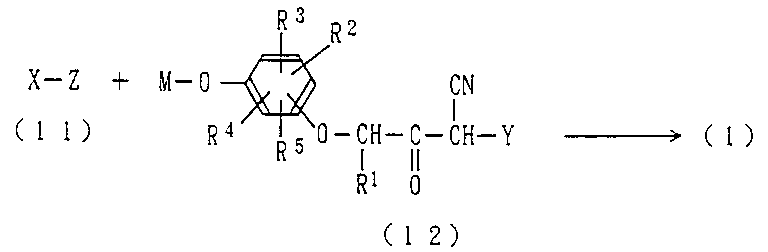 (wherein R¹, R², R³, R⁴, R⁵, X, Y, Z, and M have the same meanings as defined above).
(e): A compound represented by formula (13) and a compound represented formula (14) are reacted in the presence or absence of a solvent to obtain a ketonitrile derivative represented by formula (1) in which Y is N,N-di-substituted carbamoyl. 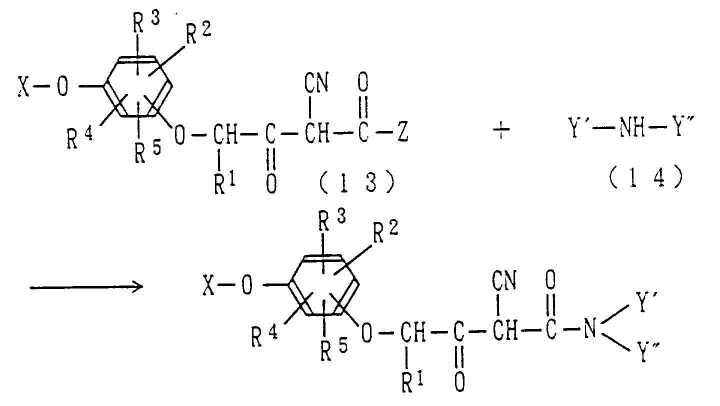 (wherein each of Y' and Y'' represents a hydrogen atom, substituted or unsubstituted alkyl, alkenyl, alkoxyl, alkylthio, alkoxycarbonyl, cyano, a substituted or unsubstituted aromatic group, or a substituted or unsubstituted heterocyclic group, in which Y' and Y'' may link to form a saturated or unsaturated ring which may contain a heterologous atom, and R¹, R², R³, R⁴, R⁵, X, Y, and Z have the same meanings as defined above).

Solvents which are used in the above methods (a) to (e) are not particularly limited, and any known solvents may be used. Typical examples of solvents which are generally used as suitable ones include alcohols such as methanol and ethanol; ethers such as diethylether, dimethoxyethane, tetrahydrofuran, and dioxane; aromatic hydrocarbons such as benzene and toluene; chlorine-containing solvents such as methylene chloride, chloroform, and carbon tetrachloride; N,N-dimethylformamide, dimethylsulfoxide, and sulforane.

It is preferred that, if necessary, a hydrogen halide scavenger be present in the reaction system in order to remove hydrogen halide that occurred as a byproduct. The hydrogen halide scavenger is not particularly limited, and conventional ones can be used. Typical and preferable examples of the hydrogen halide scavenger include trialkylamines such as triethylamine, trimethylamine, and tripropylamine; pyridine, sodium alcoholato, potassium alcoholato, DBU, sodium carbonate, potassium carbonate, sodium hydroxide, potassium hydroxide, and sodium hydride.

The reactions in methods (a) to (e) are preferably carried out at a temperature between -30°C and 200°C, and preferably between 5°C and 150°C. The reaction time is from 0.5 to 45 hours, and preferably 3 to 24 hours.

The ketonitrile derivatives (1) are isolated from the reaction mixture and purified by known methods, which are not particularly limited. In usual circumstances, the following procedure is preferably performed: The reaction mixture is first combined with water, followed by being taken up in an organic solvent and subsequent removal of the solvent. The residue is purified by recrystallization or column chromatography.

Generally speaking, the ketonitrile derivatives which are used in the present invention are pale yellow or yellowish brown viscous matter or solid matter at room temperature and atmospheric pressure.

The antibacterial agent of the present invention may be formed into various dosage forms and applied by various administration methods in accordance with the species of subjects to which the agent is administered, i.e., mammals including humans, poultry, fish, etc.

When humans are the subjects, the agent of the present invention is administered orally or parenterally. In this case, the agent may be arbitrarily formulated into preparations for oral use such as tablets, powders, granules, capsules, liquids, syrups, elixirs, and oil-base or aqueous suspensions; injections; preparations for topical use such as liquids, suspensions, emulsions, ointments, gels, creams, lotions, and sprays; and suppositories.

If the agent is formed into solid preparations, pharmaceutically acceptable carriers for medicines, such as fillers, bulking agents, binders, disintegrants, solubilizers, wetting agents, excipients, and lubricants may be used.

If the agent is formed into injections, the injection products may contain stabilizers, preservatives, tonicity agents, and solution adjuvants. Moreover, the injection preparations, after having been put in containers, may be freeze-dried to provide solid products. They will be returned to injection liquids upon use. Although a single dosage may be contained in a single container, it is also possible that a plurality of dosages may be contained in a single container.

When liquid preparations are formulated, they may contain suspending agents, emulsifying agents, or the like.

In order to administer the antibacterial agent of the present invention to mammals other than humans, poultry, or fish, the agent may be directly applied orally or may be combined with or dissolved in feed or drinking water. Alternatively, the agent may be parenterally dosed by means of injection, per rectum, mammary injection, or medicated bath (in the case of fish). For these purposes, the agent may be suitably formed, for example, into powders, granules, soluble powders, syrups, liquids, injections, suppositories, and mammary injections as desired.

The dosage of the antibacterial agent of the present invention is 50 mg to 1 g, preferably 100 mg to 600 mg, per day for adult in cases of humans. When the agent is administered to mammals other than humans, poultry, or fish, the dosage differs depending on the purpose of administration (therapy or prophylactic treatment), species and size of the subject to be treated, types of bacteria which caused infection, and severity of the infection. Generally, the agent is administered in an amount of 1-200 mg/day, preferably 5-100 mg/day per kg body weight at a single dose or in 2-4 divided doses each day. For any case of humans, animals, and others, the dosage can be modified according to the conditions of the morbid conditions, age, and the body weight.

The antibacterial agent of the present invention exhibits enhanced antibacterial activities against bacteria which belong to the genus *Staphylococcus*, *Streptococcus*, *Listeria*, *Bacillus*, *Clostridium*, *Corynebacteirum*, *Erysipelothrix*, *Bordetella*, and *Pasteurella*.

Accordingly, the antibacterial agent of the present invention is effectively applicable to various diseases caused by the above bacteria including, for example, folliculitis, furuncle, carbuncle, erysipelas, lymphangitis/lymphonoditis, abscess, hidradenitis, anal abscess, mastitis, superficial secondary infections associated with wounds, burns, or operative wounds, bronchitis, pneumonia, pyelonephritis, cystitis, urethritis, intrauterine infections, otitis media, sinuitis, endocarditis, sepsis, anthrax, and tetanus. The agent of the invention is also effectively applicable to diseases of various infections of non-human animals including, for example, diseases of staphylococcus and Clostridium infections of poultry, Streptococcus infections of pigs, swine erysipelas, Corynebacteirum infections and Clostridium infections of pigs, mastitis of cows, cattle Clostridium infections, cystitis and uretine abscess of dogs and cats, Streptococcus infections and Enterococcus infections of fish, and pseudoluberculosis of fish.

### Examples:

The present invention will next be described by way of examples, which should not be construed as limiting the invention.

### Preparation Example 1:

### Preparation of 4-[4-(3-chloro-5-trifluoromethyl-2-pyridyloxy)phenoxy]-2-(2,4-dichlorophenyl)-3-oxopentanonitrile (Compound No. 2):

A potassium salt (3.2 g) of 2-(2,4-dichlorophenyl)-4-(4-hydroxyphenoxy)-3-oxopentanonitrile and 2,3-dichloro-5-trifluoromethylpyridine (1.8 g) in N,N-dimethylformamide (50 ml) were heated at 50°C for 4 hours. Subsequently, N,N-dimethylformamide was distilled off under reduced pressure. The residue was combined with water, and then subjected to extraction with chloroform. The extract was concentrated. The residue was separated and purified by column chromatography. As a result, the title compound was obtained as a white solid (yield 60%).

### Preparation Example 2:

### Preparation of 4-[4-(2-chloro-4-trifluoromethylphenoxy)phenoxy]-2-methylphenylamidecarbonile-3-oxopentanitrile (Compound No. 36):

3,4-Dichlorobenzotrifluoride (2 g), 4-[4-(4-hydroxyphenoxy)phenoxy]-2-methylphenylamidecarbonile-3-oxopentanitrile (3 g) and potassium carbonate (3 g) in dimethylformamide (50 ml) were heated at 160°C for 6 hours. The reaction mixture was filtered and the filtrate was removed. The residue was separated and purified by column chromatography. As a result, the title compound was obtained as a pale yellow viscous material (4.00 g, yield 83.2%).

### Preparation Example 3:

### Preparation of 4-[4-(3-chloro-5-trifluoromethylpyridyloxy)phenoxy]-2-dibenzylamidecarbonile-3-oxopentanitrile (Compound No. 38):

2-[4-(2-Chloro-5-trifluoromethyl-2-pyridyloxy)phenoxy propionyl chloride (3.00 g) in benzene (20 ml) was added dropwise to a mixture solution of N,N-dibenzyl-2-cyanoacetamide (1.20 g), DBU (1.44 g), and benzene (30 ml). The thus-obtained mixture was refluxed at 90°C for 5 hours. The reaction mixture was discarded and subjected to extraction with chloroform. The extract was concentrated. The residue was separated and purified by column chromatography. As a result, the title compound was obtained as a pale yellow viscous material (2.08 g, yield 53.1%).

### Preparation Example 4:

In a manner similar to those described in Preparation Example Nos. 1 to 3, compounds identified by Compound Nos. 1 to 47 were prepared.

The chemical structure of Compound Nos. 1 to 47 including the compounds obtained in Preparation Examples 1 to 3 is represented by the following formula.

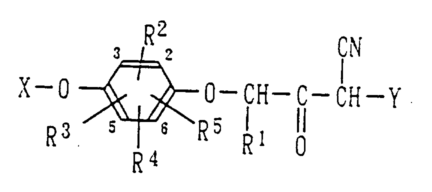

The analytical data obtained with respect to these compounds are shown in Tables 1 to 10, in which substituents R¹, R², R³, R⁴, R⁵, X, and Y are also tabulated. In the Tables, the IR spectral data are characteristic absorption values based on ether bond and cyano group, and the mass spectral data are of the molecular ion peaks (M⁺) and fragments. The numbers indicating the positions of substitution of R² through R⁵ in Tables 1 to 10 are as shown in the benzene ring of the above formula, and as will be noted, these numbers do not correspond to the numbers in the nomenclature of compounds.

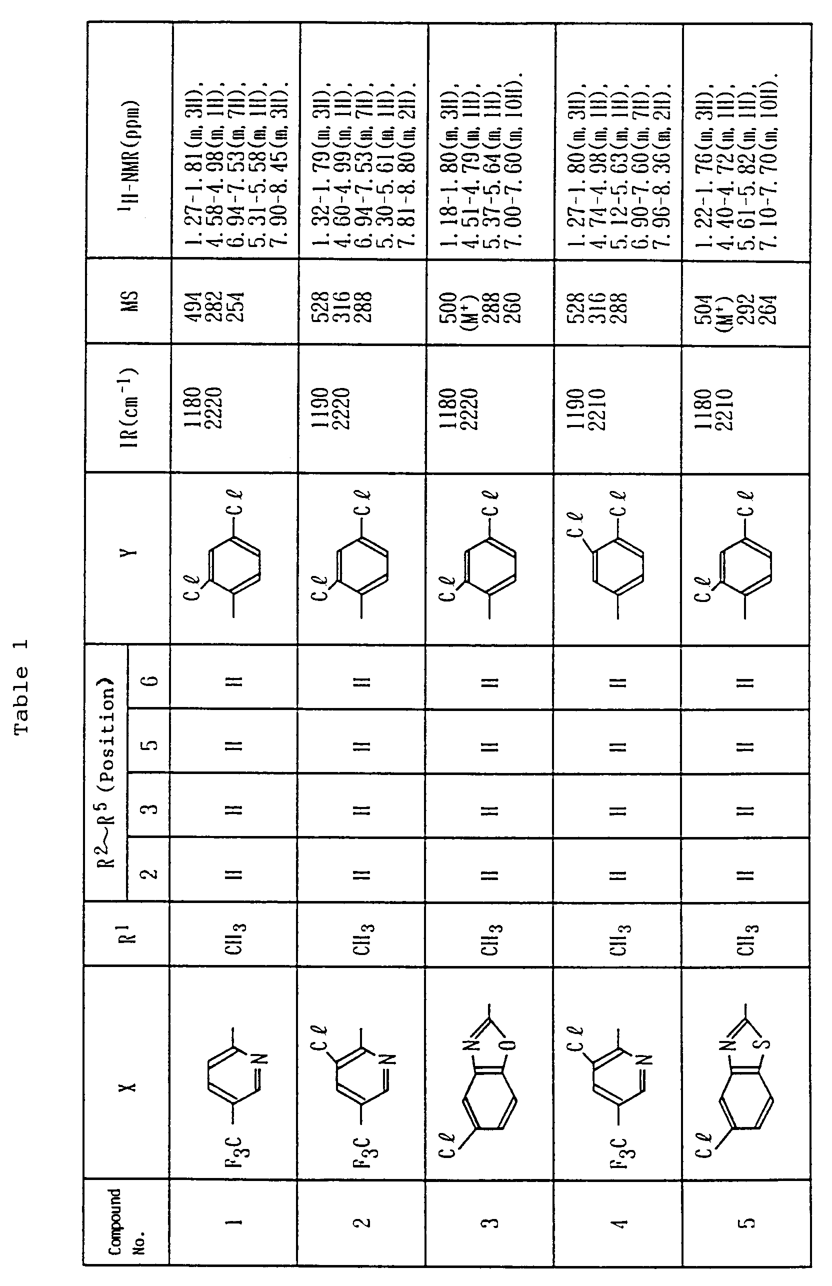

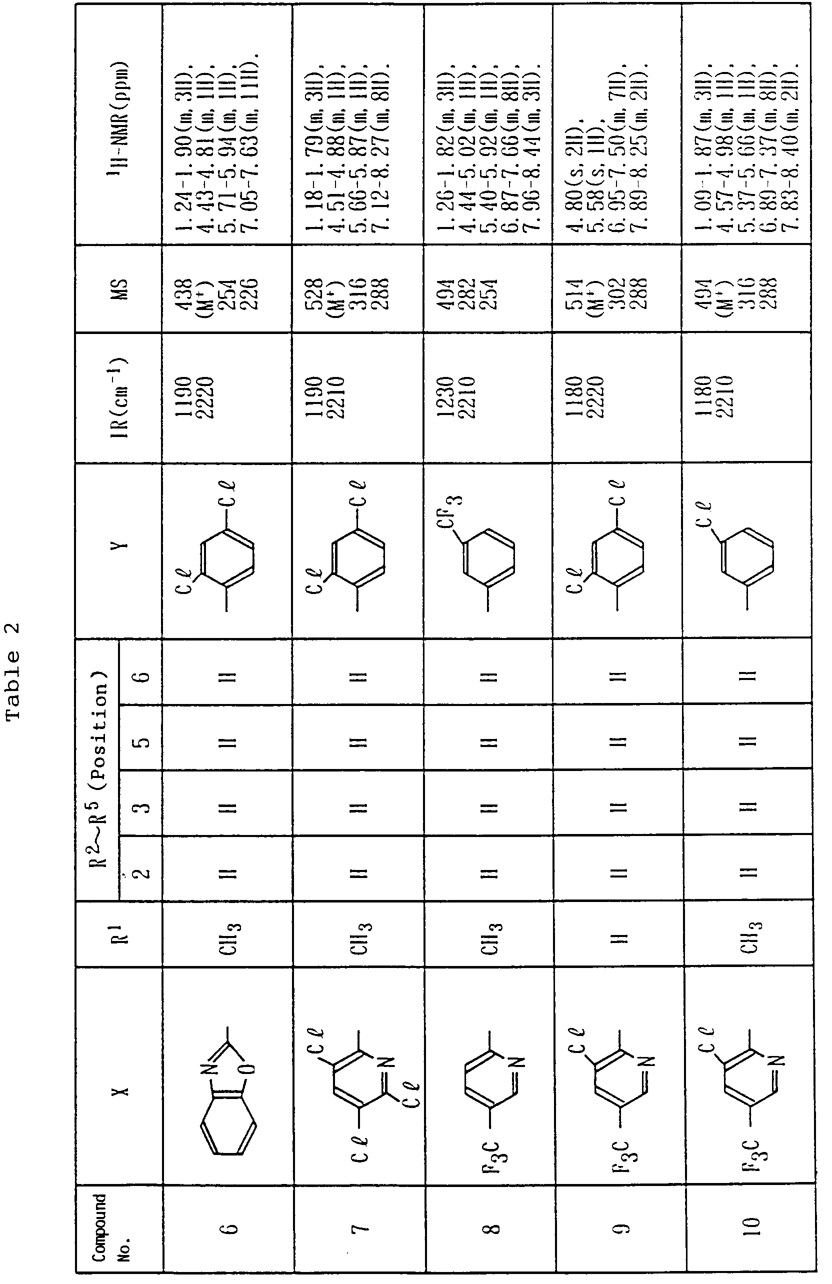

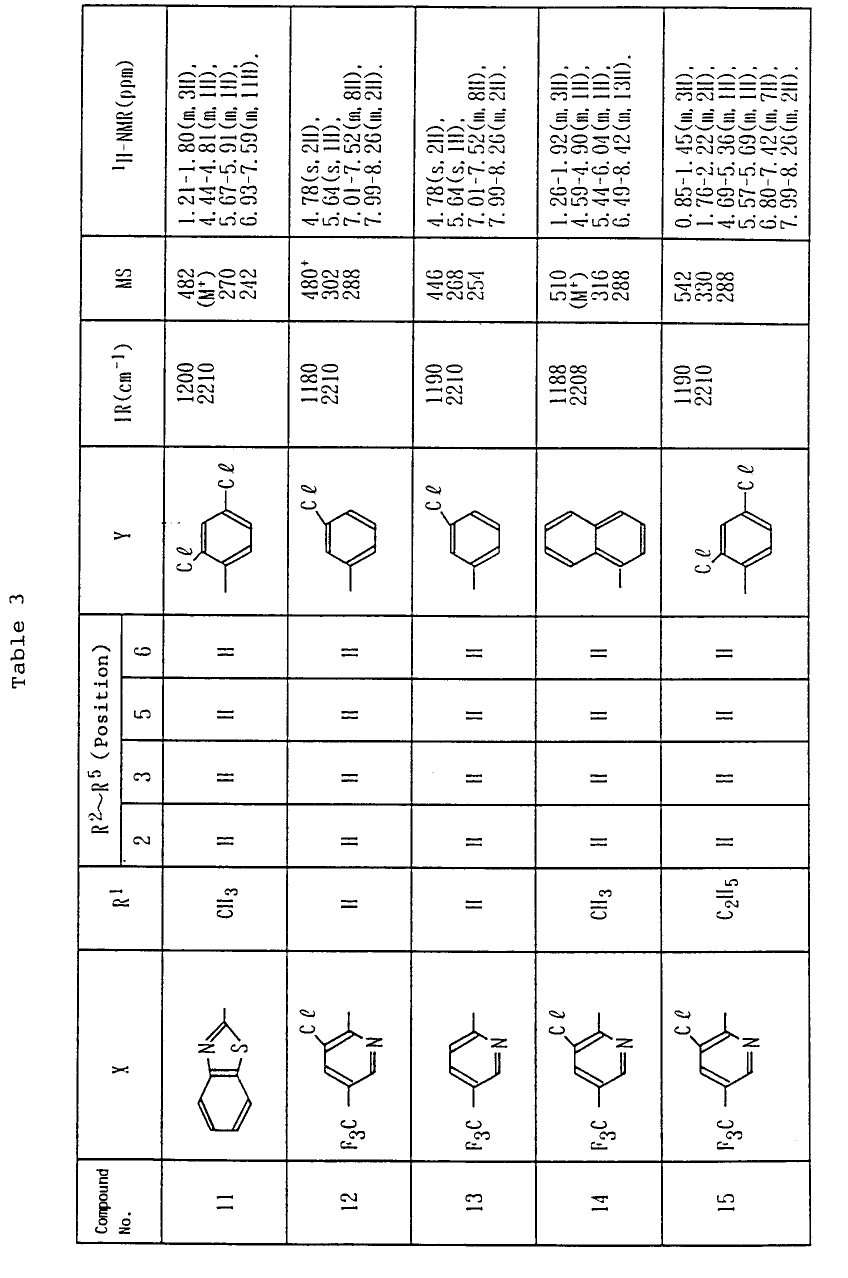

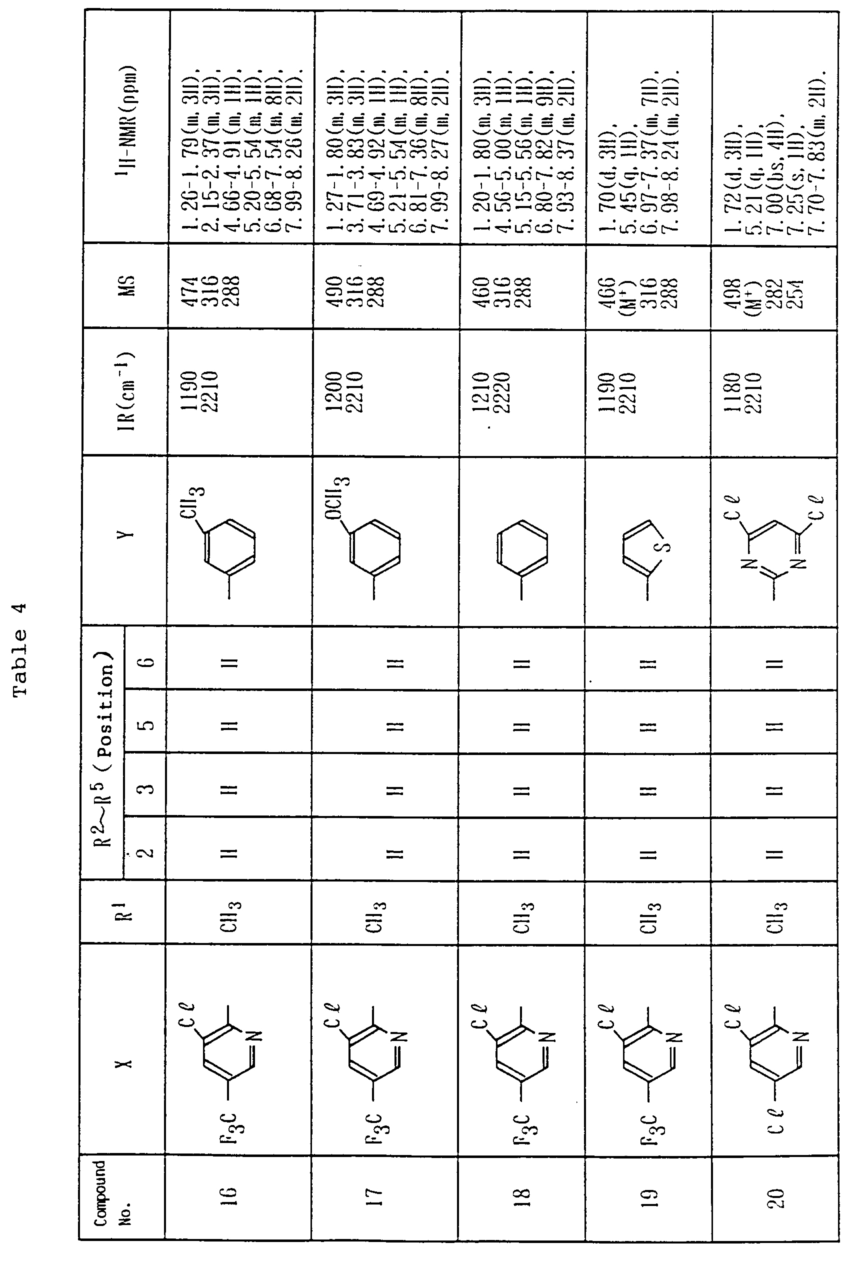

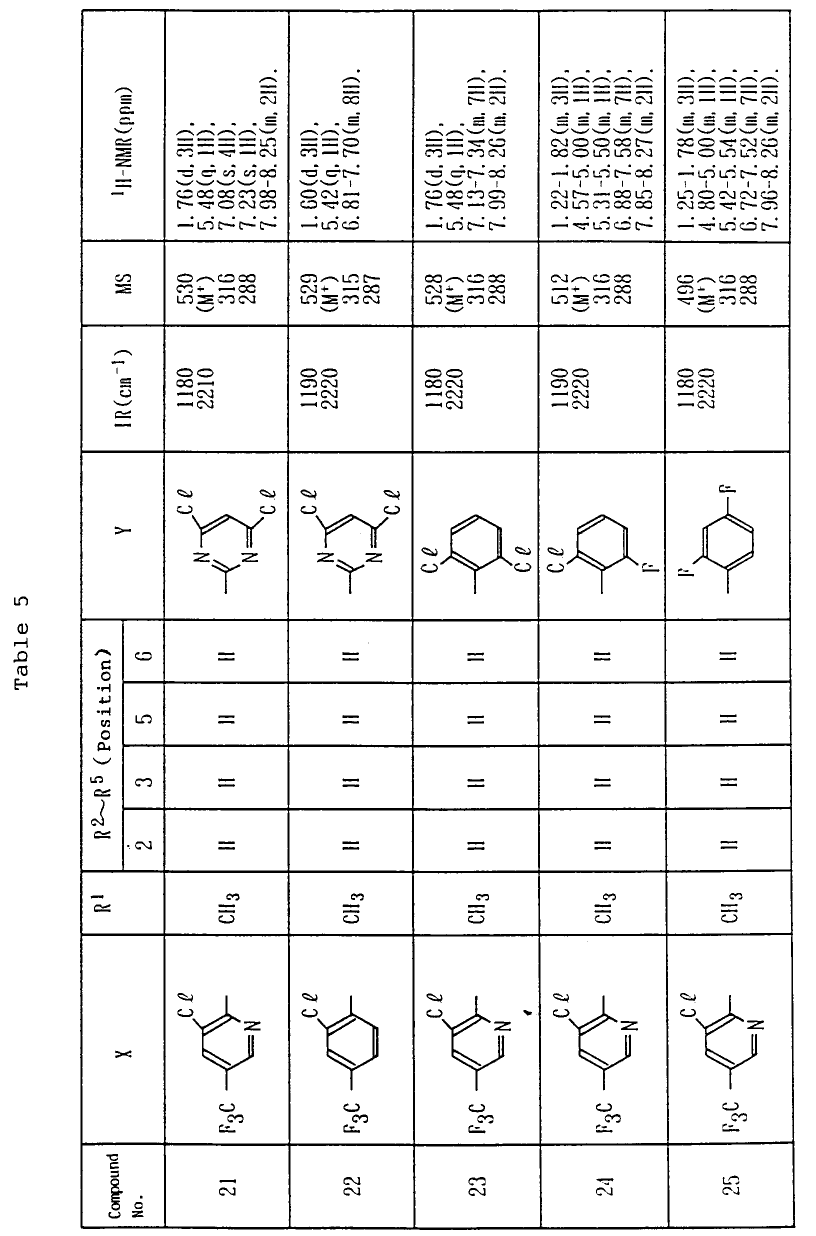

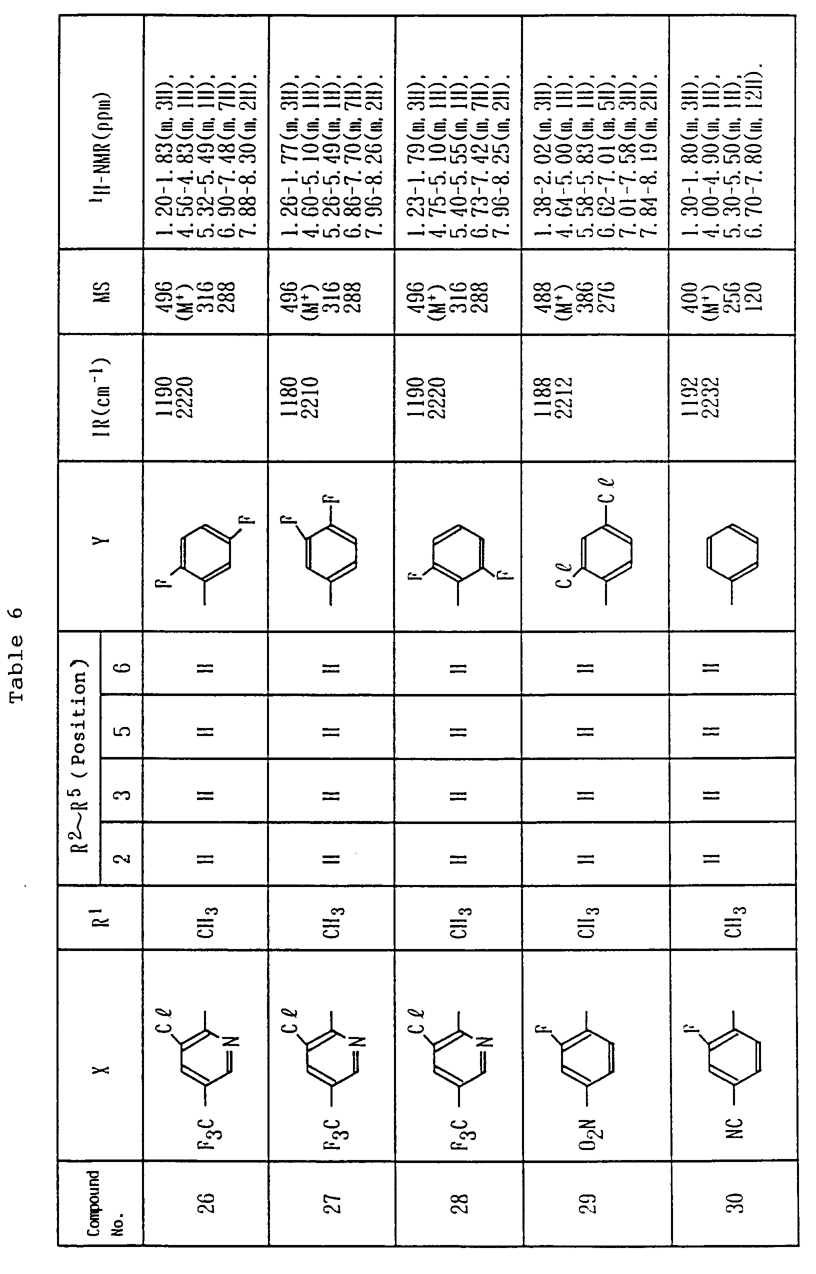

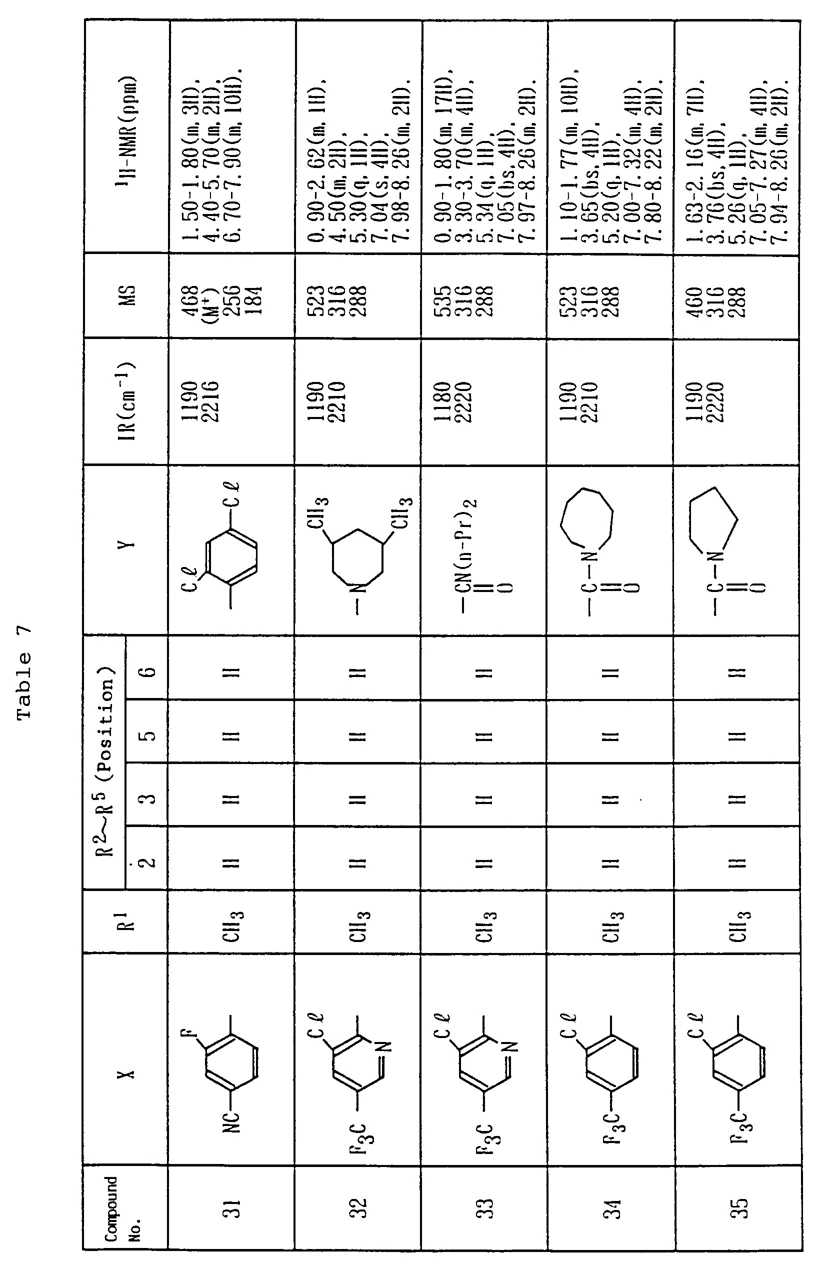

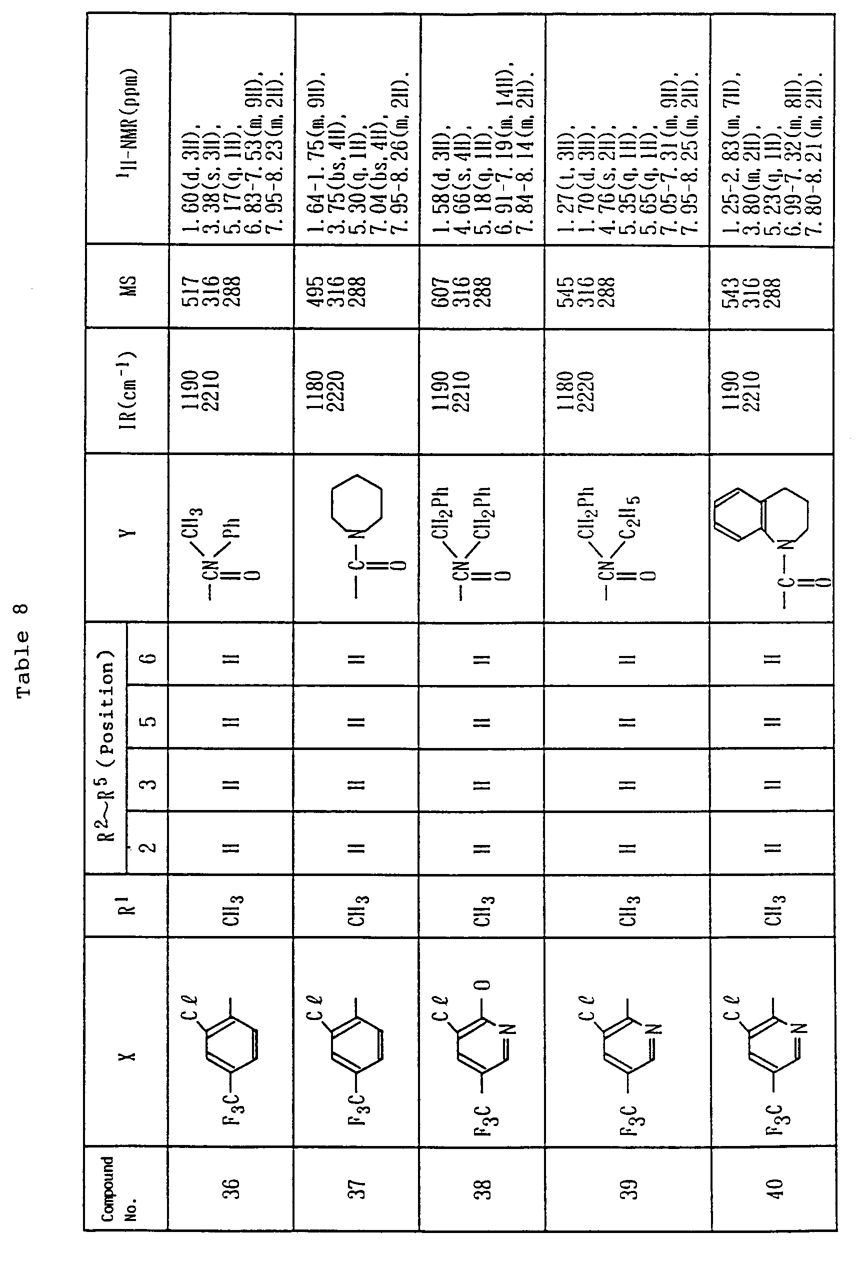

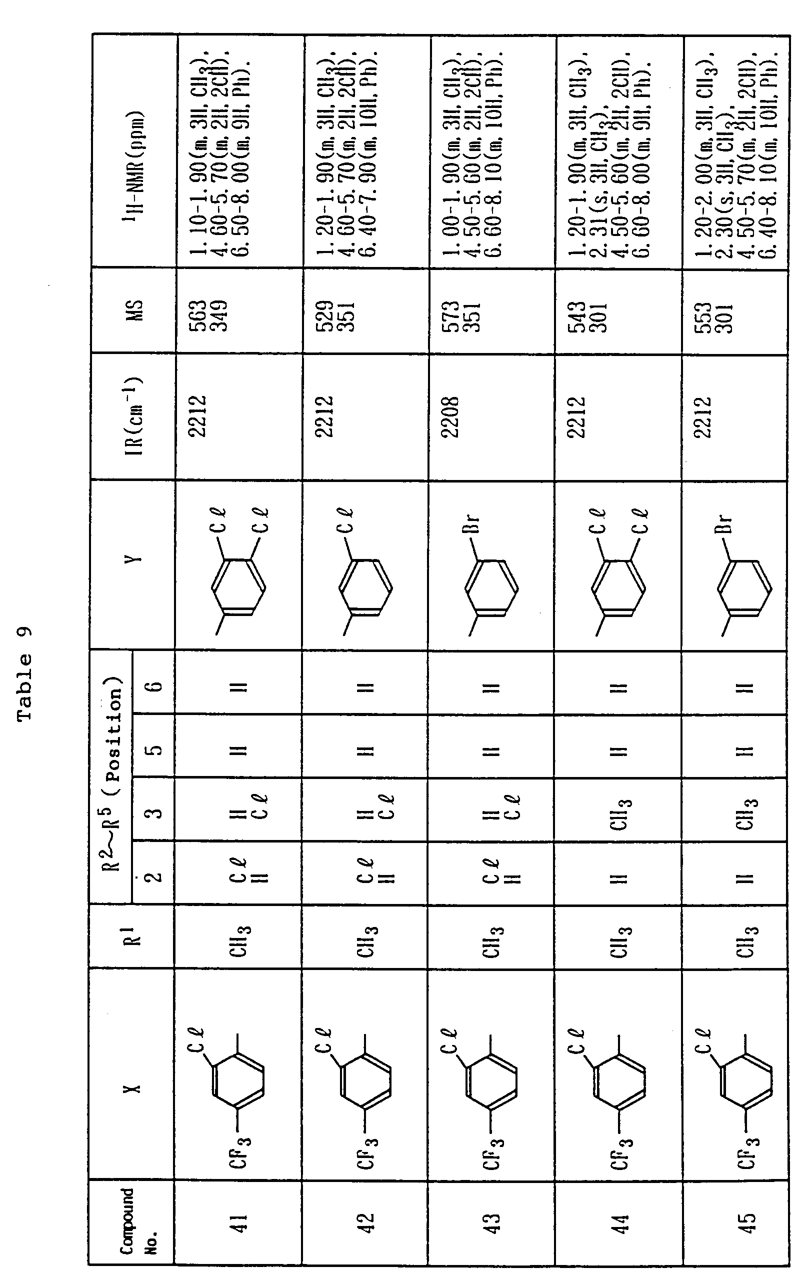

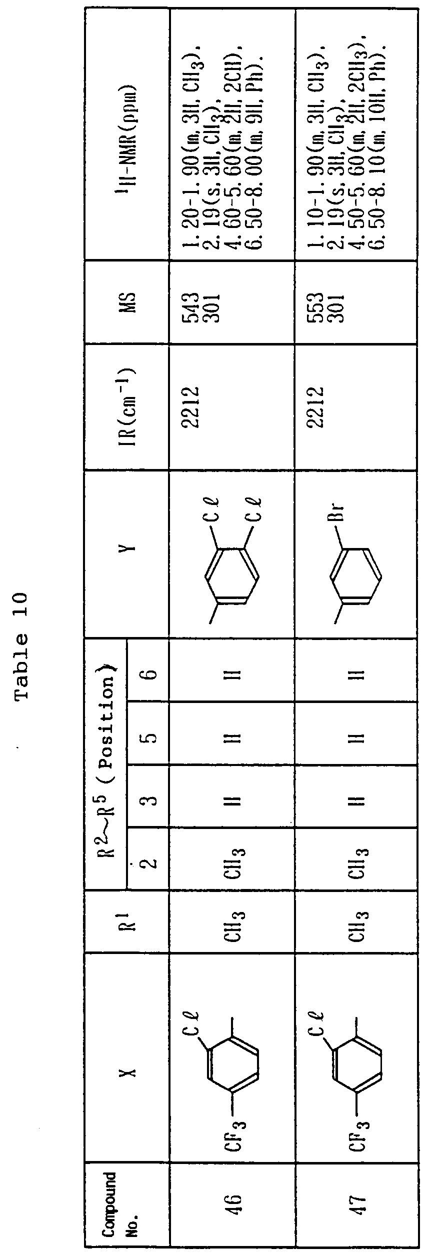

Preparation Example 5:
(1) Methyl 2-(3-hydroxyphenoxy)propionate (8 g) and 3,4-dichlorobenzyl cyanide (8.4 g) were dissolved in ethanol (100 ml). To the resulting solution was added dropwise sodium ethoxide prepared from ethanol (15 ml) and metal sodium (3.1 g). After completion of addition, the mixture was refluxed for 3 hours, and ethanol was removed by evaporation. The mixture was cooled to room temperature, and water (100 g) was added to the mixture. Through use of hydrochloric acid, the pH of the mixture was made acidic, after which extraction was performed with ethyl acetate. The organic layer was washed with water and saturated NaCl solution in this order, and dried over anhydrous magnesium sulfate. Ethyl acetate was distilled off under reduced pressure. Purification by column chromatography yielded 6.8 g of 2-(3,4-dichlorophenyl)-4-(3-hydroxyphenoxy)-3-oxopentanonitrile (48%).
(2) The thus-obtained 2-(3,4-dichlorophenyl)-4-(3-hydroxyphenoxy)-3-oxopentanonitrile (2 g) was dissolved in dimethylformamide (DMF, 30 ml). To the resulting mixture, potassium carbonate (1.6 g) and 2,3-dichloro-5-trifluoromethylpyridine (1.3 g) were added, and then the mixture was stirred at 120°C for 2 hours. Subsequently, the solution was cooled to room temperature. To the cooled solution, ether (150 ml) was added. The mixture was sequentially washed with diluted HCl, water, and saturated NaCl solution in this order, and dried over anhydrous magnesium sulfate. Ether was distilled off under reduced pressure. Purification by column chromatography yielded 1 g of 4-[3-(3-chloro-5-trifluoromethyl-2-pyridyloxy)phenoxy]-2-(3,4-dichlorophenyl)-3-oxopentanonitrile (32%) (Compound No. 101).

### Preparation Example 6:

Through use of the 2-(3,4-dichlorophenyl)-4-(3-hydroxyphenoxy)-3-oxopentanonitrile obtained in Preparation Example 5(1) and 2-chloro-5-trifluoromethylpyridine, the procedure of Preparation Example 5 was repeated. As a result, 1 g of 2-(3,4-dichlorophenyl)-3-oxo-4-[3-(5-trifluoromethyl-2-pyridyloxy)phenoxy]pentanonitrile was obtained (32%) (Compound No. 102).

### Preparation Example 7:

(1) Sodium hydride (oily, 60%) (2.6 g) was placed in a flask. By the use of hexane, the content of the flask was repeatedly decanted to remove oil components. Dimethylformamide (DMF, 50 ml) was added to the flask to suspend sodium hydride. To the suspension, 3-(2-chloro-4-trifluoromethylphenoxy)phenol (16.9 g) was added gradually. The resulting mixture was combined with methyl 2-chloropropionate (8.6 g) and then with potassium iodide (0.5 g). The mixture was stirred with heating at 80°C for 2 hours. Subsequently, it was cooled to room temperature. Ether (250 ml) was added. The mixture was washed with diluted HCl, water, and saturated NaCl solution in this order, and the ether layer was dried over anhydrous magnesium sulfate. Ether was distilled off under reduced pressure. Purification by column chromatography yielded 20.6 g of methyl 2-[3-(2-chloro-4-trifluoromethylphenoxy)phenoxy]propionate (93%).
(2) The thus-obtained methyl 2-[3-(2-chloro-4-trifluoromethylphenoxy)phenoxy]propionate and 3,4-dichlorobenzyl cyanide (1.5 g) were dissolved in ethanol (10 ml). To the resulting solution, sodium ethoxide prepared from ethanol (5 ml) and metal sodium (0.3 g) was added dropwise. The solution was refluxed for 2 hours, and then stirred overnight at room temperature. Ether (150 ml) was added. The mixture was washed with diluted HCl, water, and saturated NaCl solution in this order, and dried over anhydrous magnesium sulfate. Ether was distilled off under reduced pressure. Purification by column chromatography yielded 0.9 g of 4-[3-(2-chloro-4-trifluoromethylphenoxy)phenoxy]-2-(3,4-dichlorophenyl)-3-oxopentanonitrile (33%) (Compound No. 103).

### Preparation Example 8:

Through use of the methyl 2-[3-(2-chloro-4-trifluoromethylphenoxy)phenoxy]propionate (2 g) obtained in Preparation Example 7(1) and 3-bromobenzyl cyanide (1.3 g), the procedure of Preparation Example 7 was repeated. As a result, 0.6 g (20%) of 2-(3-bromophenyl)-4-[3-(2-chloro-4-trifluoromethylphenoxy)phenoxy]-3-oxopentanonitrile was obtained (Compound No. 104).

### Preparation Example 9:

Phenylacetonitrile (0.7 g) in tetrahydrofuran (THF) was added dropwise to lithium diisopropylamide (2 M, a heptane/THF/ethylbenzene solution) (3 ml) dissolved in THF (10 ml) at room temperature. To the resulting mixture, methyl 2-[3-(2-chloro-4-trifluoromethylphenoxy)phenoxy]propionate (2 g) in THF was added dropwise, and the mixture was stirred at room temperature for 6 hours. Ether (150 ml) was added. The mixture was sequentially washed with diluted HCl, water, and saturated NaCl solution in this order, and dried over anhydrous magnesium sulfate. Ether was distilled off under reduced pressure. Purification by column chromatography yielded 0.8 g (31%) of 4-[3-(2-chloro-4-trifluoromethylphenoxy)phenoxy]-3-oxo-2-phenylpentanonitrile (Compound No. 105).

### Preparation Example 10:

Through use of 3-trifluoromethylbenzyl cyanide (1 g) and methyl 2-[3-(2-chloro-4-trifluoromethylphenoxy)phenoxy] propionate (2 g), the procedure of Preparation Example 9 was repeated. As a result, 0.8 g (31%) of 4-[3-(2-chloro-4-trifluoromethylphenoxy)phenoxy]-3-oxo-2-(3-trifluoromethylphenyl)pentanonitrile was obtained (Compound No. 106).

### Preparation Example 11:

Through use of 4-methoxybenzyl cyanide (0.9 g) and methyl 2-[3-(2-chloro-4-trifluoromethylphenoxy)-phenoxy]propionate (2 g), the procedure of Preparation Example 9 was repeated. As a result, 0.8 g (29%) of 4-[3-(2-chloro-4-trifluoromethylphenoxy)-phenoxy]-2-(4-methoxyphenyl)-3-oxopentanonitrile was obtained (Compound No. 107).

### Preparation Example 12:

Through use of 2,4-dichlorobenzyl cyanide (1.1 g) and methyl 2-[3-(2-chloro-4-trifluoromethylphenoxy)-phenoxy]propionate (2 g), the procedure of Preparation Example 7 was repeated. As a result, 0.6 g (21%) of 4-[3-(2-chloro-4-trifluoromethyl-phenoxy)phenoxy]-2-(2,4-dichlorophenyl)-3-oxopentanonitrile was obtained (Compound No. 108).

### Preparation Example 13:

Through use of 3-(3-bromo-2-fluorophenoxy)phenol (5.5 g) and methyl 2-chloropropionate (3.3 g), methyl 2-[3-(4-bromo-2-fluorophenoxy)phenoxy]propionate (6.9 g) was prepared (96%). By the use of the thus-obtained methyl 2-[3-(4-bromo-2-fluorophenoxy)-phenoxy]propionate (1 g) and 3,4-dichlorobenzyl cyanide (0.7 g), the procedure of Preparation Example 7 was repeated. As a result, 0.2 g (17%) of 4-[3-(4-bromo-2-fluorophenoxy)phenoxy]-2-(3,4-dichlorophenyl)-3-oxopentanonitrile was obtained (Compound No. 109).

### Preparation Example 14:

Through use of methyl 2-[3-(4-bromo-2-fluorophenoxy)phenoxy]-propionate (2 g) and 3-chlorobenzyl cyanide (1 g), the procedure of Preparation Example 7 was repeated. As a result, 0.6 g (23%) of 4-[3-(4-bromo-2-fluorophenoxy)phenoxy]-2-(3-chlorophenyl)-3-oxopentanonitrile was obtained (Compound No. 110).

### Preparation Example 15:

Through use of methyl 2-[3-(4-bromo-2-fluorophenoxy)phenoxy]propionate (2 g) and 3-bromobenzyl cyanide (1 g), the procedure of Preparation Example 7 was repeated. As a result, 0.6 g (21%) of 4-[3-(4-bromo-2-fluorophenoxy)phenoxy]-2-(3-bromophenyl)-3-oxopentanonitrile was obtained (Compound No. 111).

### Preparation Example 16:

Through use of 5-methylresorcinol (11 g) and 3,4-dichlorobenzotrifluoride (17 g), 3-(2-chloro-4-trifluoromethylphenoxy)-5-methylphenol (2.3 g) was synthesized (9%). Subsequently, by the use of this product and methyl 2-chloropropionate, 2.3 g of methyl 2-[3-(2-chloro-4-trifluoromethylphenoxy)phenoxy]propionate was synthesized (79%). Furthermore, by the use of this material and 3,4-dichlorobenzyl cyanide (1.1 g), 0.2 g (5%) of 4-[3-(2-chloro-4-trifluoromethylphenoxy)-5-methylphenoxy]-2-(3,4-dichlorophenyl)-3-oxopentanonitrile was obtained (Compound No. 112).

### Preparation Example 17:

Through use of 5-methoxyresorcinol (10 g) and 3,4-dichlorobenzotrifluoride (16 g), 3-(2-chloro-4-trifluoromethylphenoxy)-5-methoxyphenol (5.2 g) was synthesized (23%). Subsequently, by the use of this product and methyl 2-chloropropionate, 5.9 g of methyl 2-[3-(2-chloro-4-trifluoromethylphenoxy)-5-methoxyphenoxy]propionate was synthesized (86%). Furthermore, by the use of this material (2 g) and 3,4-dichlorobenzyl cyanide (0.9 g), 0.4 g (14%) of 4-[3-(2-chloro-4-trifluoromethylphenoxy)-5-methoxyphenoxy]-2-(3,4-dichlorophenyl)-3-oxopentanonitrile was obtained (Compound No. 113).

### Preparation Example 18:

Through use of 4-chlororesorcinol (15 g) and 3,4-dichlorobenzotrifluoride (19 g), 3-(2-chloro-4-trifluoromethylphenoxy)-4-chlorophenol (11 g) was synthesized (33%). Subsequently, by the use of this product and methyl 2-chloropropionate, 13 g of methyl 2-[3-(2-chloro-4-trifluoromethylphenoxy)-4-chlorophenoxy]propionate was synthesized (93%). Furthermore, by the use of this material (2 g) and 1-naphthylacetonitrile (0.8 g), 0.5 g (19%) of 4-[5-(2-chloro-4-trifluoromethylphenoxy)-2-chlorophenoxy]-2-(1-naphthyl)-3-oxopentanonitrile was obtained (Compound No. 114).

### Preparation Example 19:

Pyridine (0.79 g) was added dropwise to a mixture of 2-[5-(2-chloro-4-trifluoromethylphenoxy)-2-nitrophenoxy]propionyl chloride (2.12 g), 2-pyridylacetonitrile (0.65 g), and anhydrous toluene (20 ml). The resultant mixture was stirred for a day at room temperature. Then it was concentrated, and made acidic with aqueous 10% HCl solution. Extraction was performed with ethyl acetate. The extract was concentrated.

The residue was recrystallized from ether - ethyl acetate. As a result, 1.06 g (41.9%) of 4-[5-(2-chloro-4-trifluoromethylphenoxy)-2-nitrophenoxy]-3-oxo-2-(2-pyridyl)pentanonitrile was obtained (Compound No. 115).

### Preparation Example 20:

2,4-Dichlorophenylacetonitrile (0.93 g) in anhydrous tetrahydrofuran (THF) (30 ml) was cooled to -78°C. To this solution, 1.6 M n-butyl lithium - hexane solution (3.5 ml) was added. Subsequently, 2-[5-(2-chloro-4-trifluoromethylphenoxy)-2-nitrophenoxy]propionyl chloride (2.12 g) in THF was added to the resultant mixture dropwise. The mixture was stirred for 30 minutes. Aqueous 10% HCl solution was added to make the mixture acidic. Extraction was performed with ethyl acetate. The organic layer was dried over anhydrous magnesium sulfate and concentrated.

The concentrated residue was purified by silica gel chromatography. As a result, 0.75 g (26.3%) of 4-[5-(2-chloro-4-trifluoromethylphenoxy)-2-nitrophenoxy]-2-(2,4-dichlorophenyl)-3-oxopentanonitrile was obtained (Compound No. 116).

### Preparation Example 21:

To a solution obtained by dissolving metal sodium (0.35 g) in ethanol (50 ml), ethyl 2-[5-(2-chloro-4-trifluoromethylphenoxy)-2-nitro]propionate (2.16 g) and 2,3-dichlorophenylacetonitrile (0.93 g) were added, and the mixture was refluxed with heat for 4 hours. The reaction mixture was concentrated, and the residue was combined with aqueous 10% HCl. Extraction was performed with chloroform, and the extract was concentrated. The residue was purified by silica gel column chromatography. As a result, 1.86 g (65.0%) of 4-[5-(2-chloro-4-trifluoromethylphenoxy)-2-nitrophenoxy]-2-(3,4-dichlorophenyl)-3-oxopentanonitrile was obtained (Compound No. 117).

### Preparation Example 22:

Methyl 3-[5-(2-chloro-4-trifluoromethylphenoxy)-2-chlorophenoxy]propionate (2 g) and 3-bromobenzyl cyanide (1 g) were dissolved in ethanol. To the resultant mixture, sodium ethoxide prepared through use of ethanol and metal sodium (0.3 g) was added dropwise. The mixture was refluxed for 2 hours, and then cooled to room temperature. Ether was added. The mixture was washed with diluted HCl, water, and saturated NaCl solution in this order, and then dried over anhydrous magnesium sulfate. Magnesium sulfate was filtered off, and ether was distilled off under reduced pressure. The thus-obatined crude product was purified by column chromatography to give 0.8 g (28%) of 2-(3-bromophenyl)-4-[5-(2-chloro-4-trifluoromethylphenoxy)-2-chlorophenoxy]-3-oxopentanonitrile (Compound No. 155).

### Preparation Example 23:

To a solution of N-methylpiperazine (0.5 g), triethylamine (0.87 g), and dimethoxyethane (30 ml), 4-[5-(2-chloro-4-trifluoromethylphenoxy)-2-nitrophenoxy]-2-cyano-3-oxopentanoyl chloride (2.45 g) in dimethoxyethane (20 ml) was added dropwise. The resultant mixture was stirred at room temperature for 6 hours. The reaction mixture was discarded, and extracted with chloroform. The extract was concentrated. The residue was purified by silica gel chromatography. As a result, 2.11 g (76.2%) of 4-[5-(2-chloro-4-trifluoromethylphenoxy)-2-nitrophenoxy]-2-(4-methyl-1-piperazinylcarbonyl)-3-oxopentanonitrile was obtained (Compound No. 138).

### Preparation Example 24:

In a manner similar to that described in Preparation Examples 5 to 23, compound Nos. 118 to 217 were prepared.

The chemical structure of Compound Nos. 101 to 217 including the compounds obtained in Preparation Examples 5 to 23 is represented by the following formula.

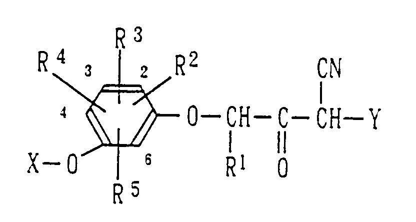

The analytical data obtained with respect to these compounds are shown in Tables 11 to 34, in which substituents R¹, R², R³, R⁴, R⁵, X, and Y are also tabulated. In the Tables, the IR spectral data are characteristic absorption values based on ether bond and cyano group, and the mass spectral data are the molecular ion peaks (M⁺) and fragments. The numbers indicating the positions of substitution of R² through R⁵ in Tables 11 to 34 are shown in the benzene ring of the above formula, and as will be noted, these numbers do not correspond to the numbers in the nomenclature of compounds.

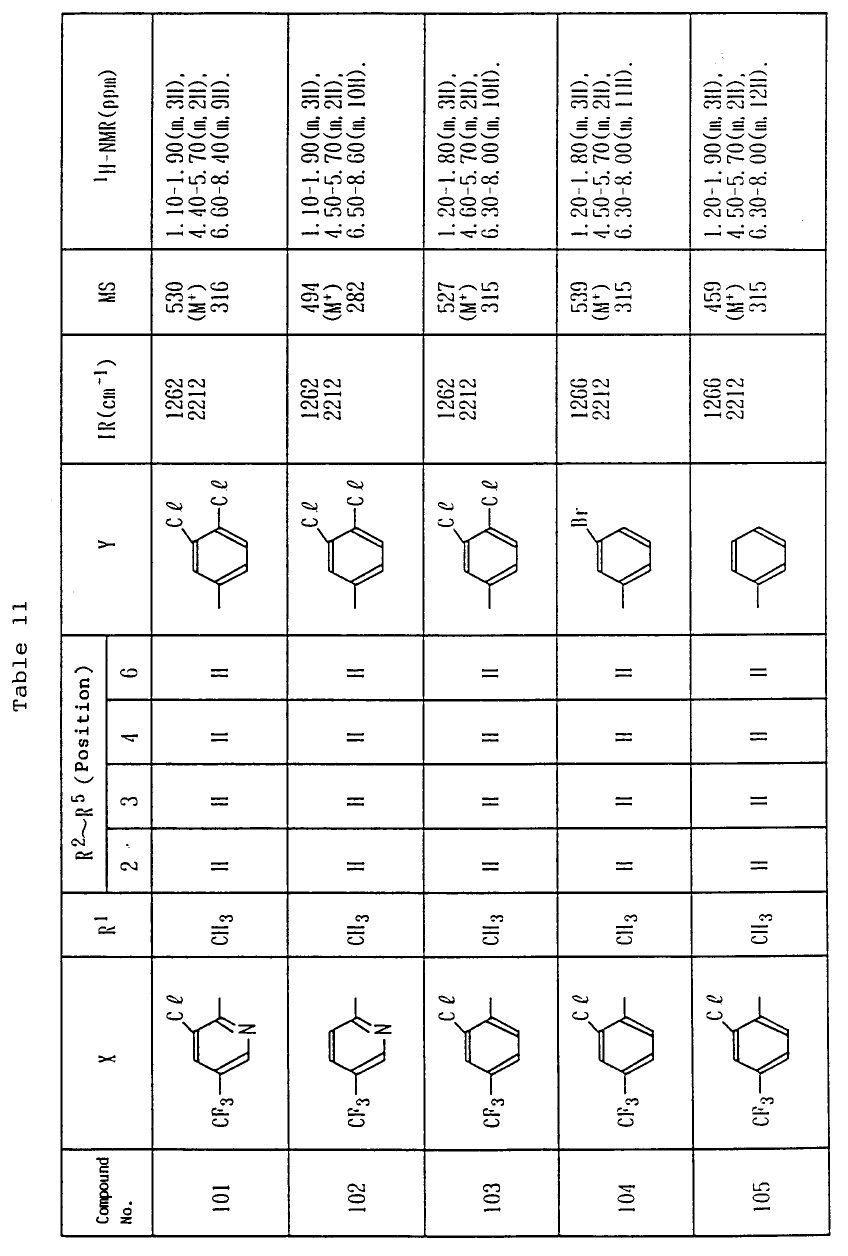

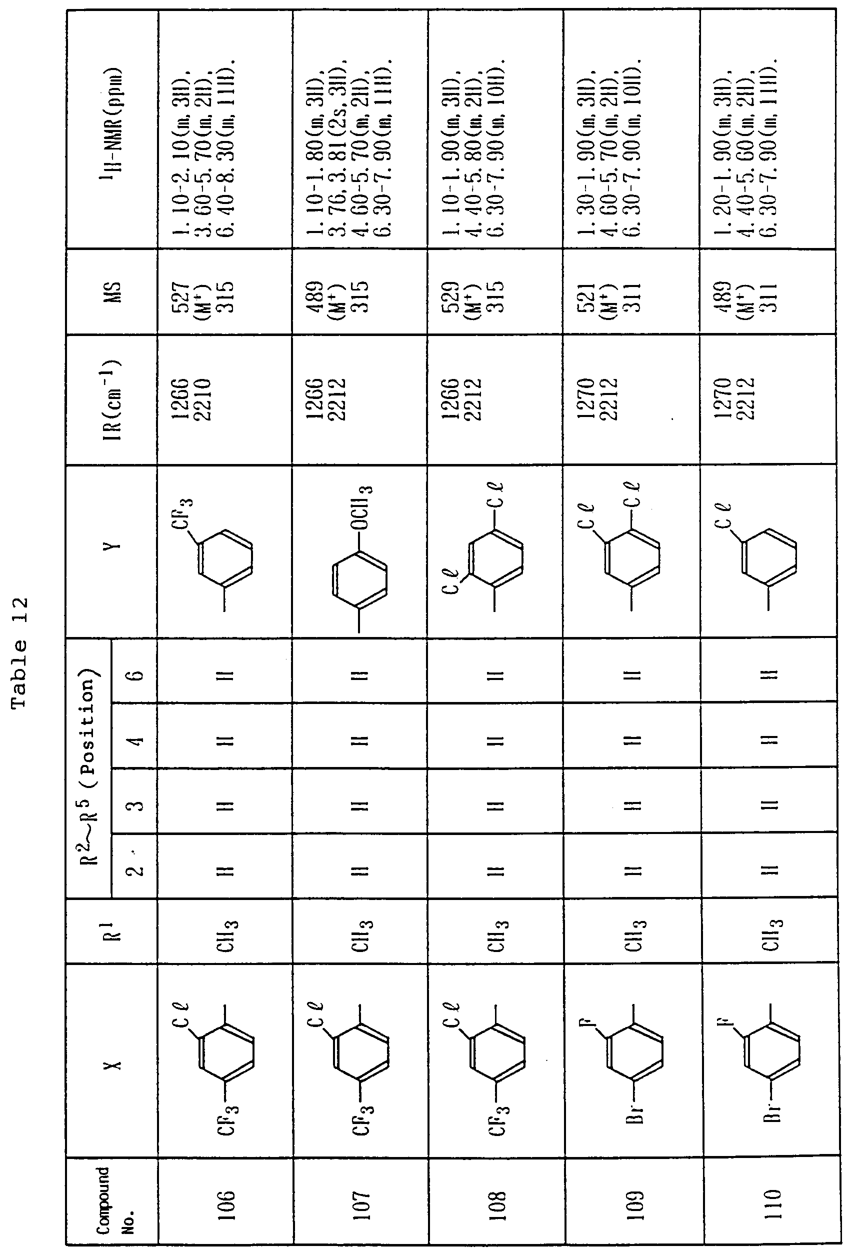

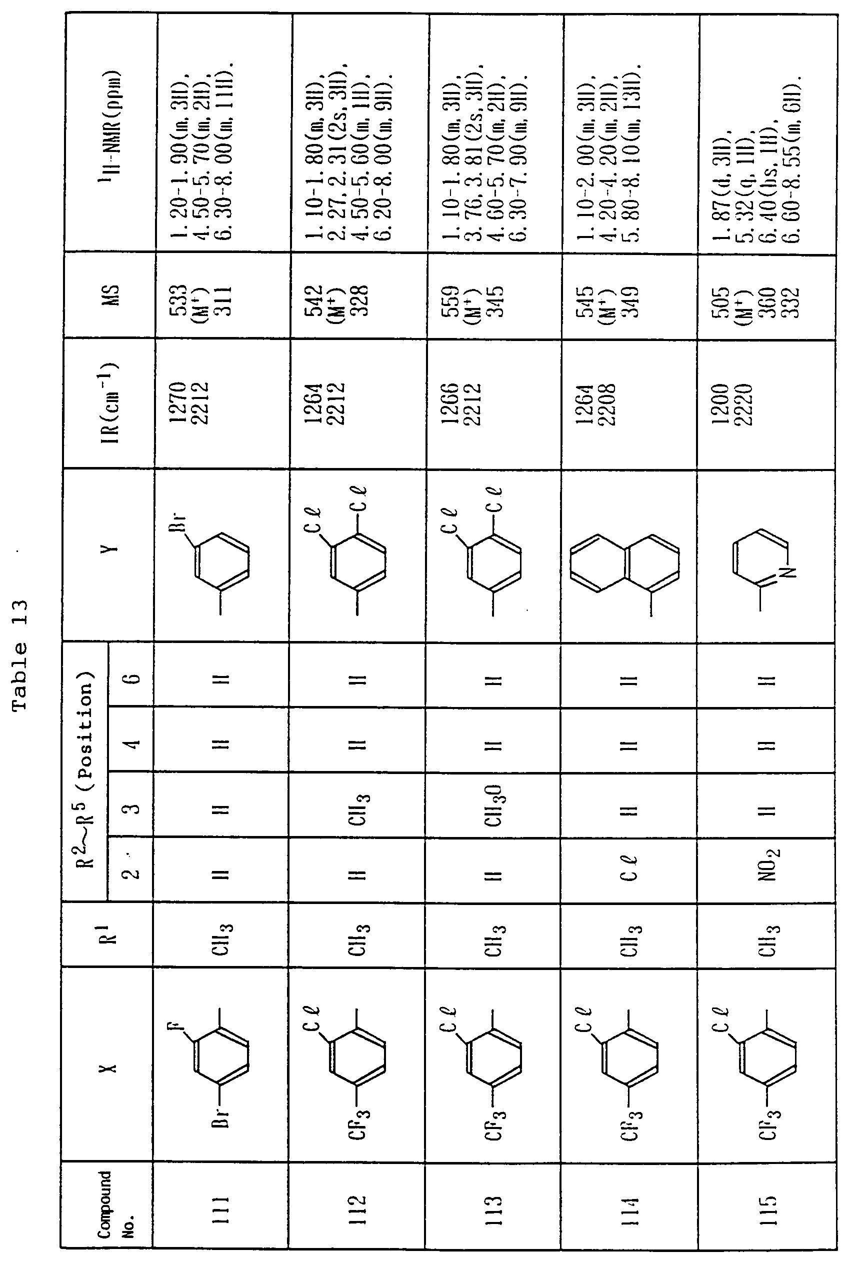

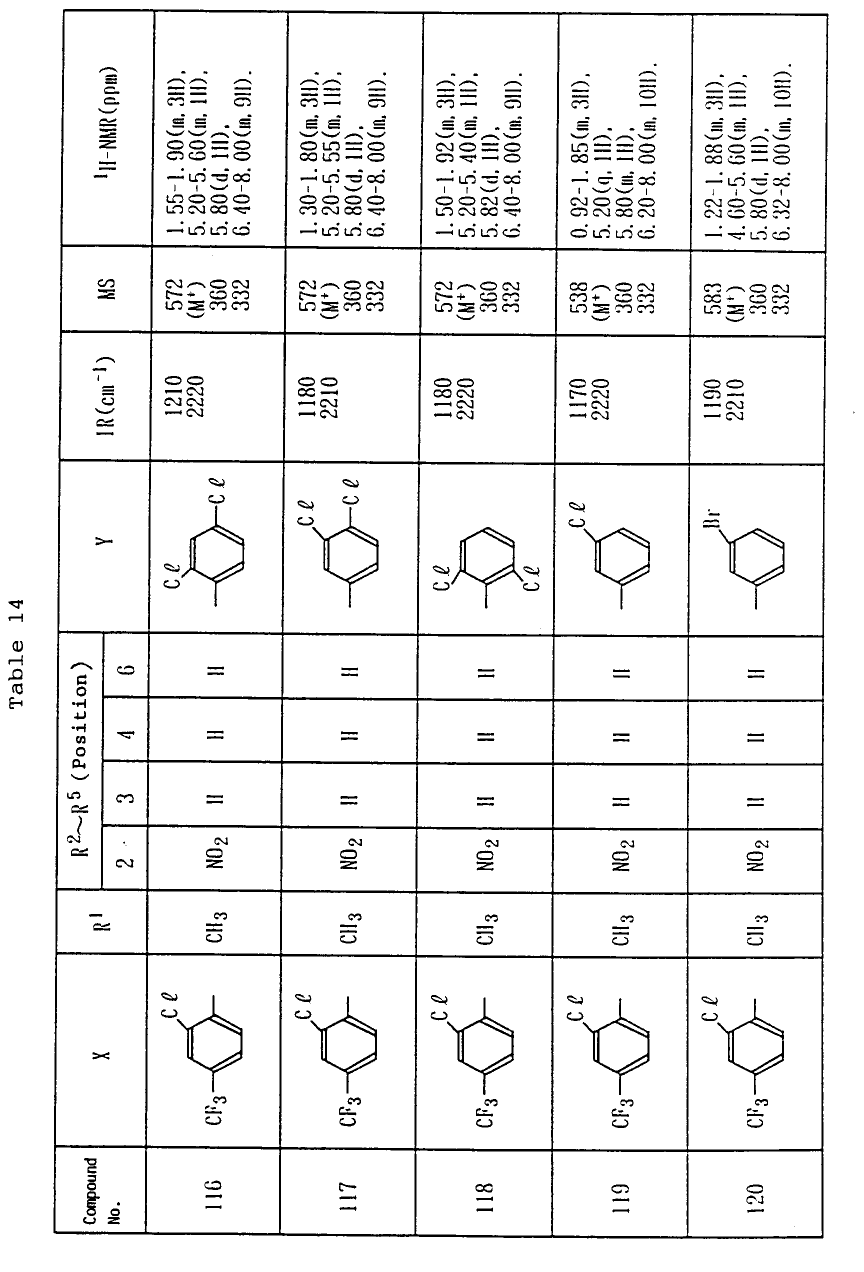

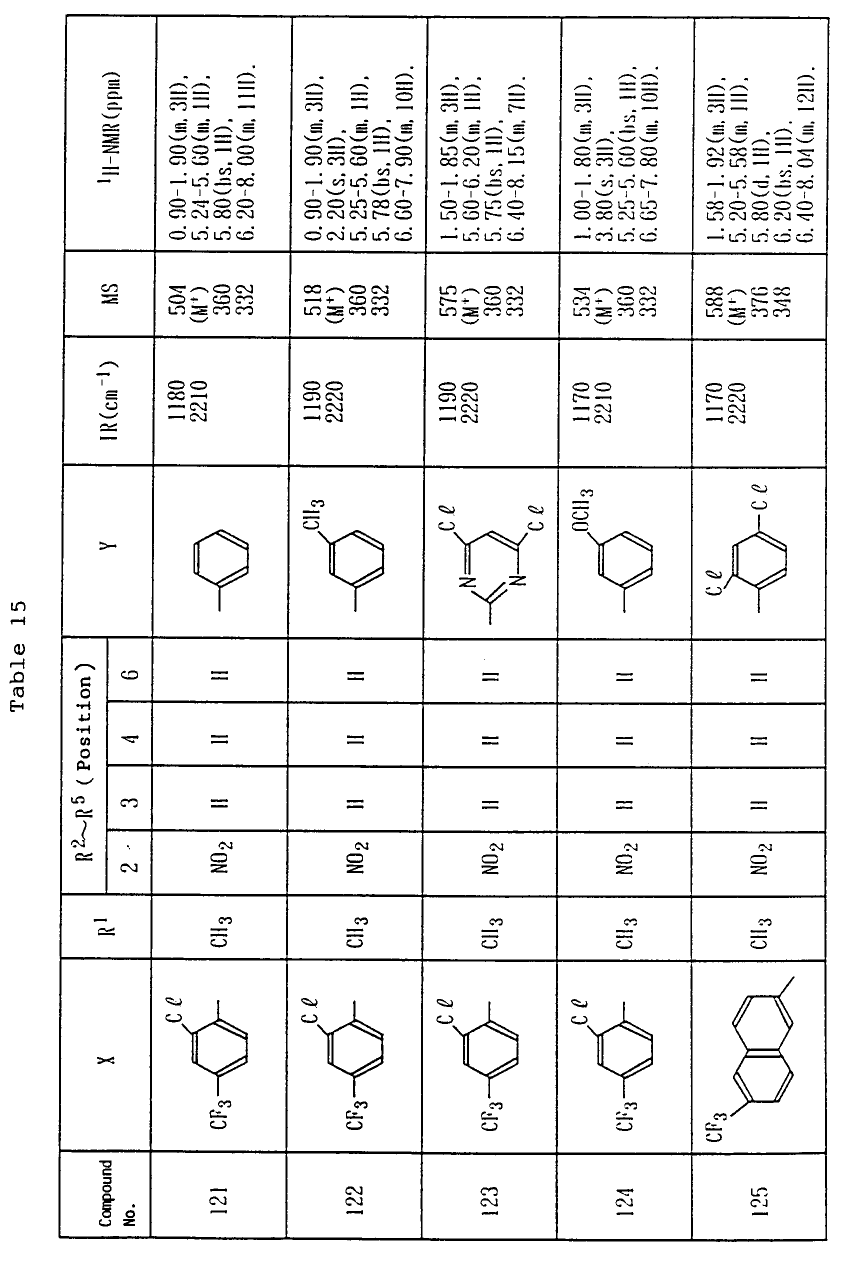

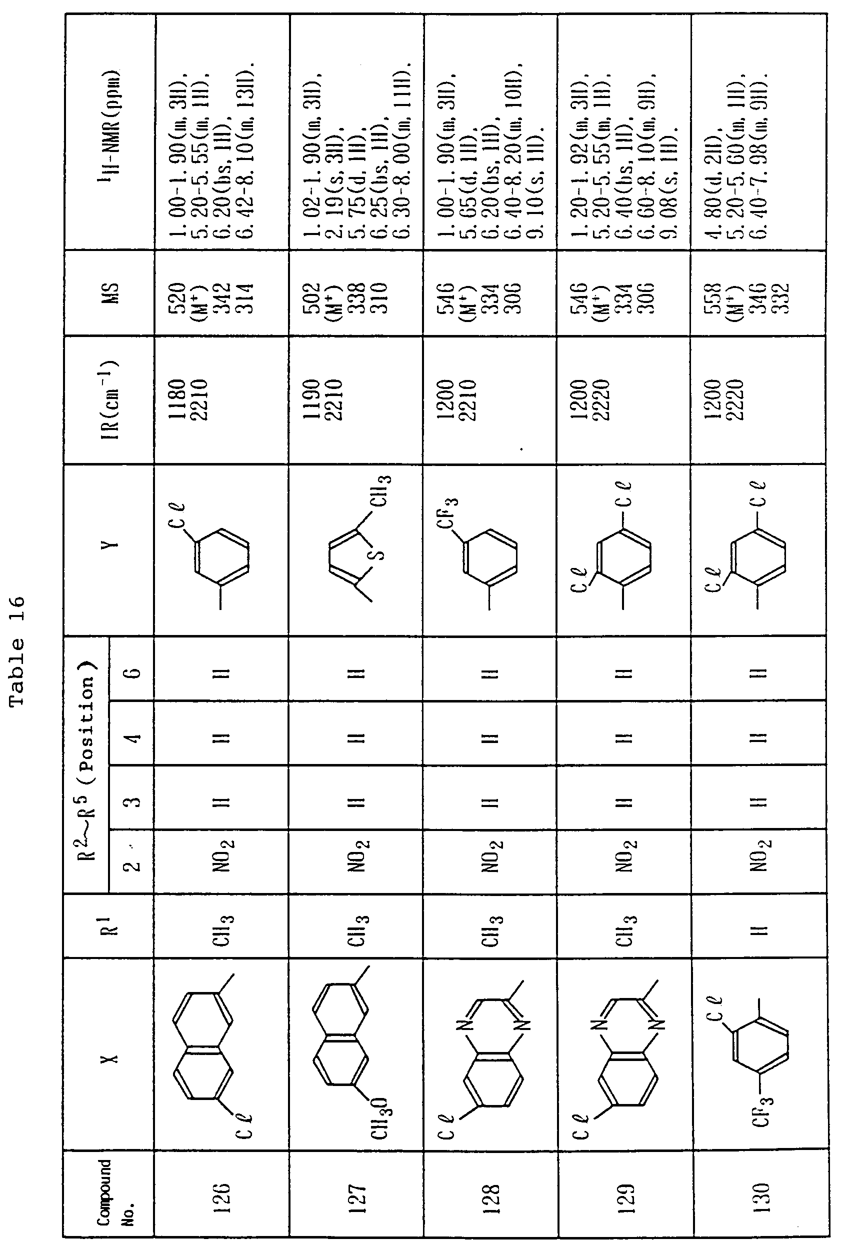

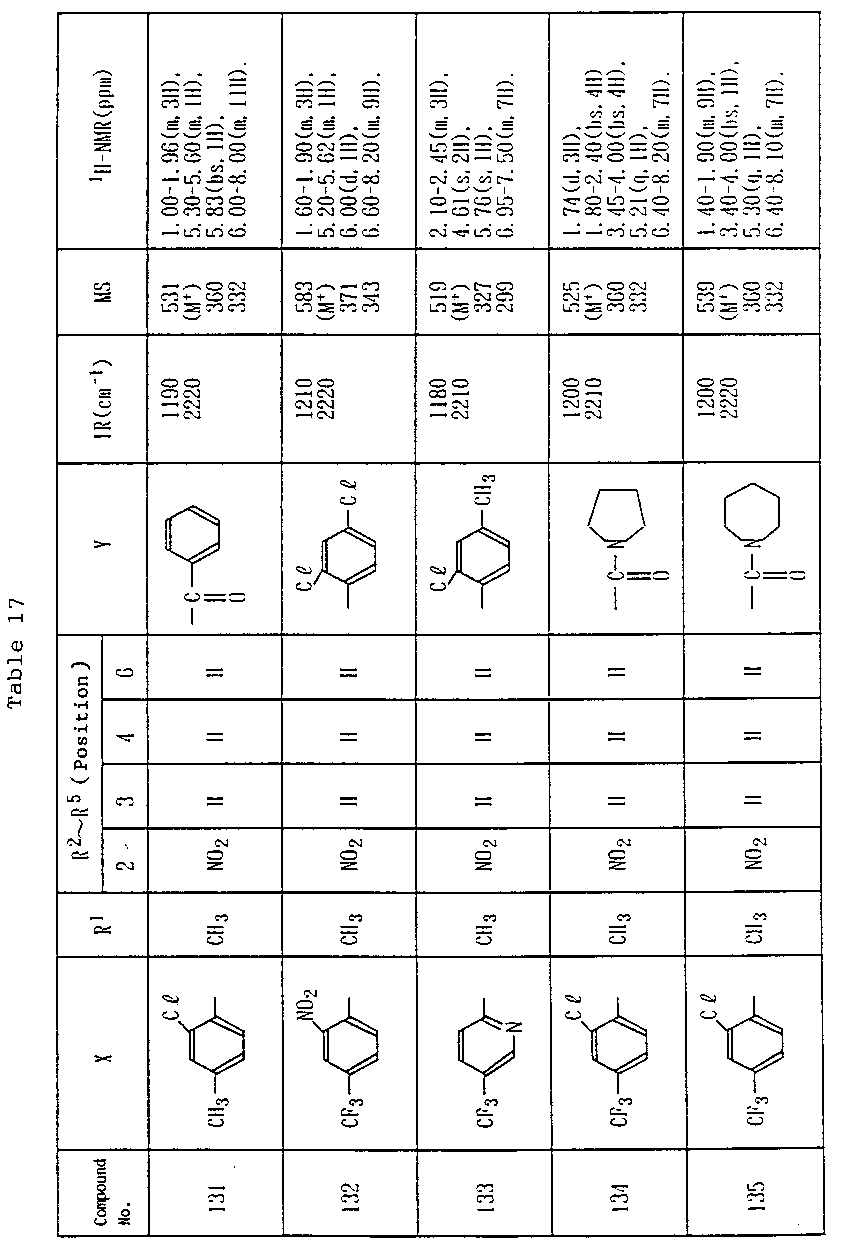

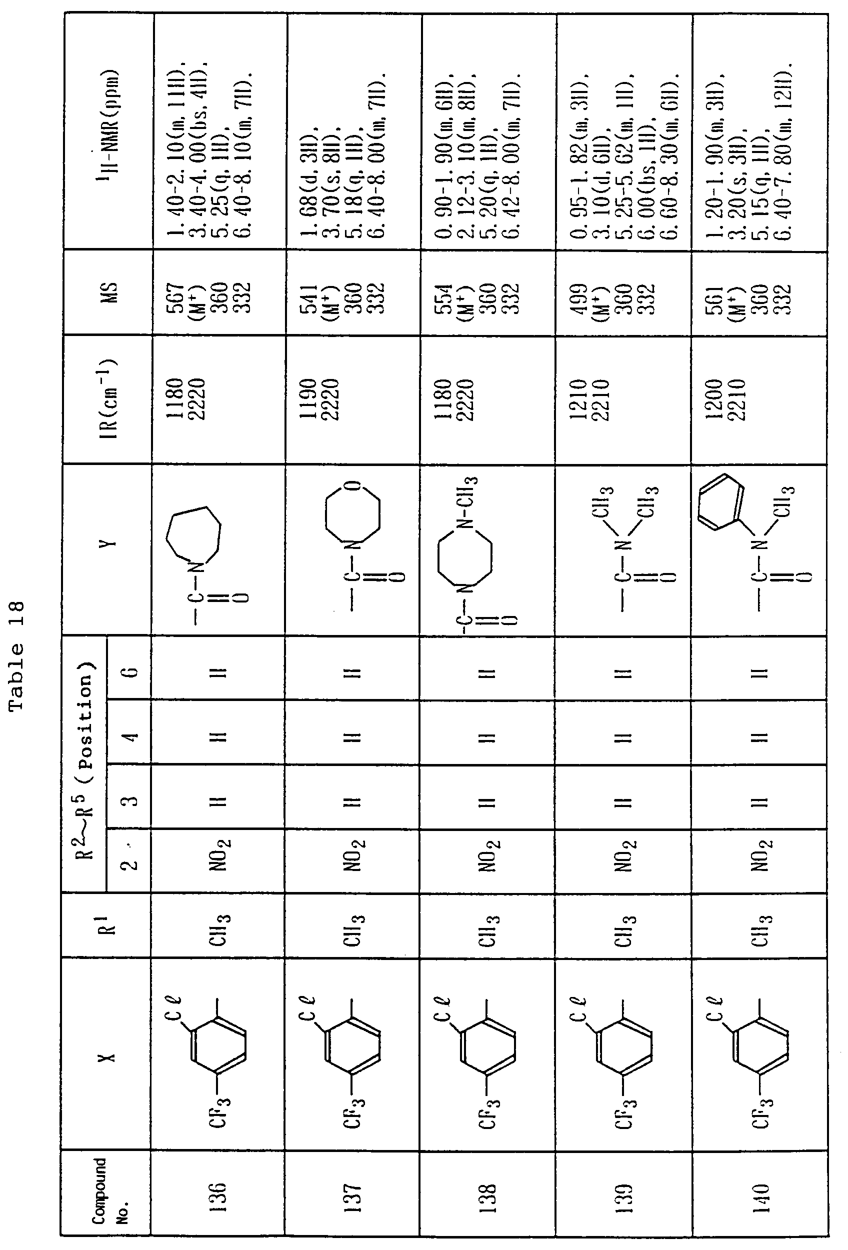

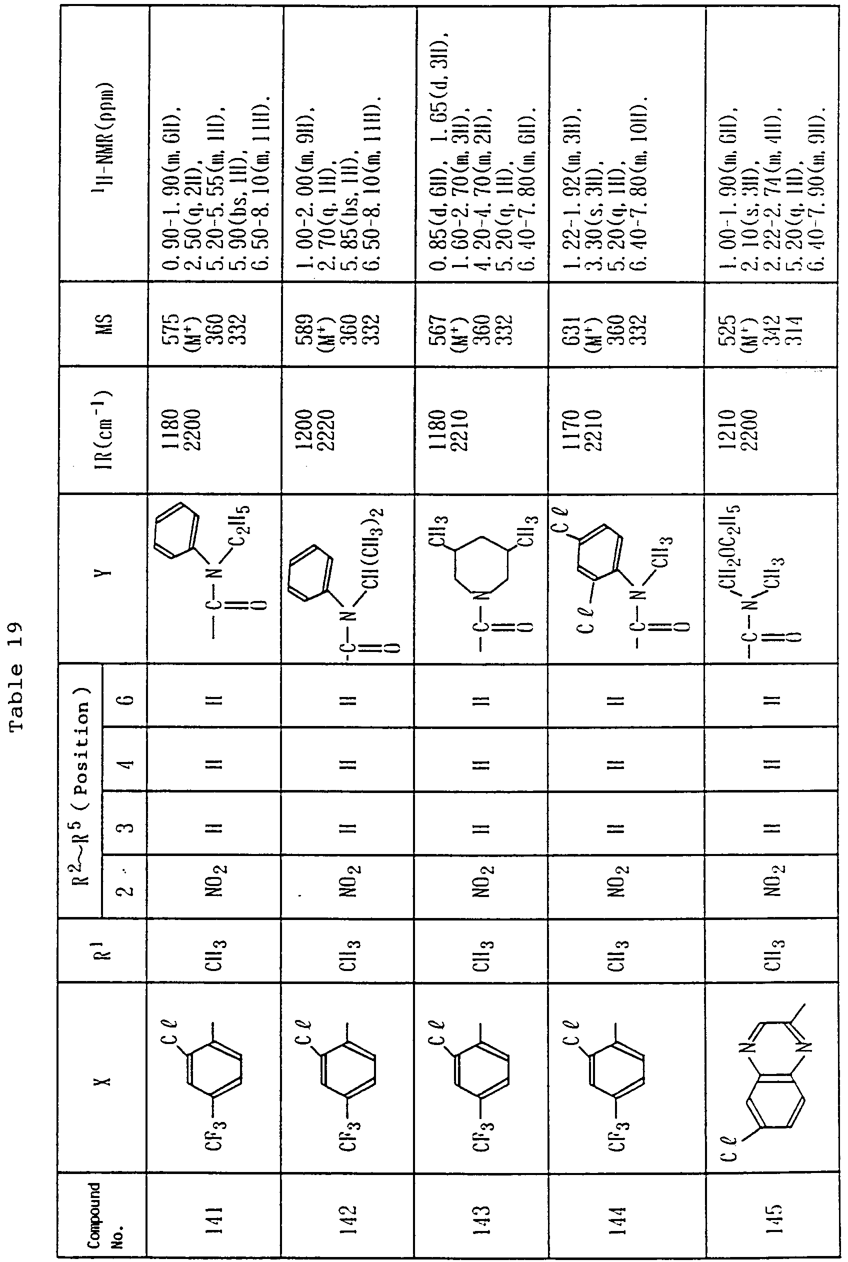

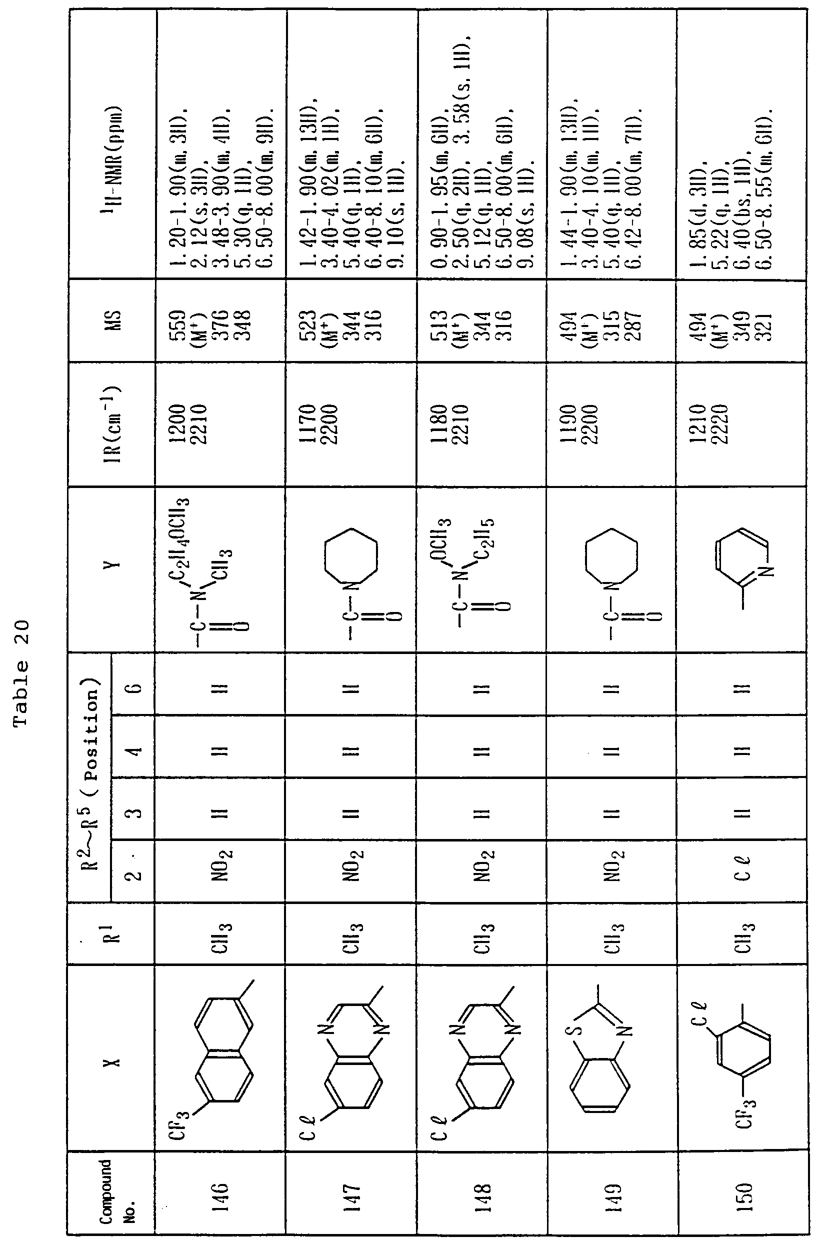

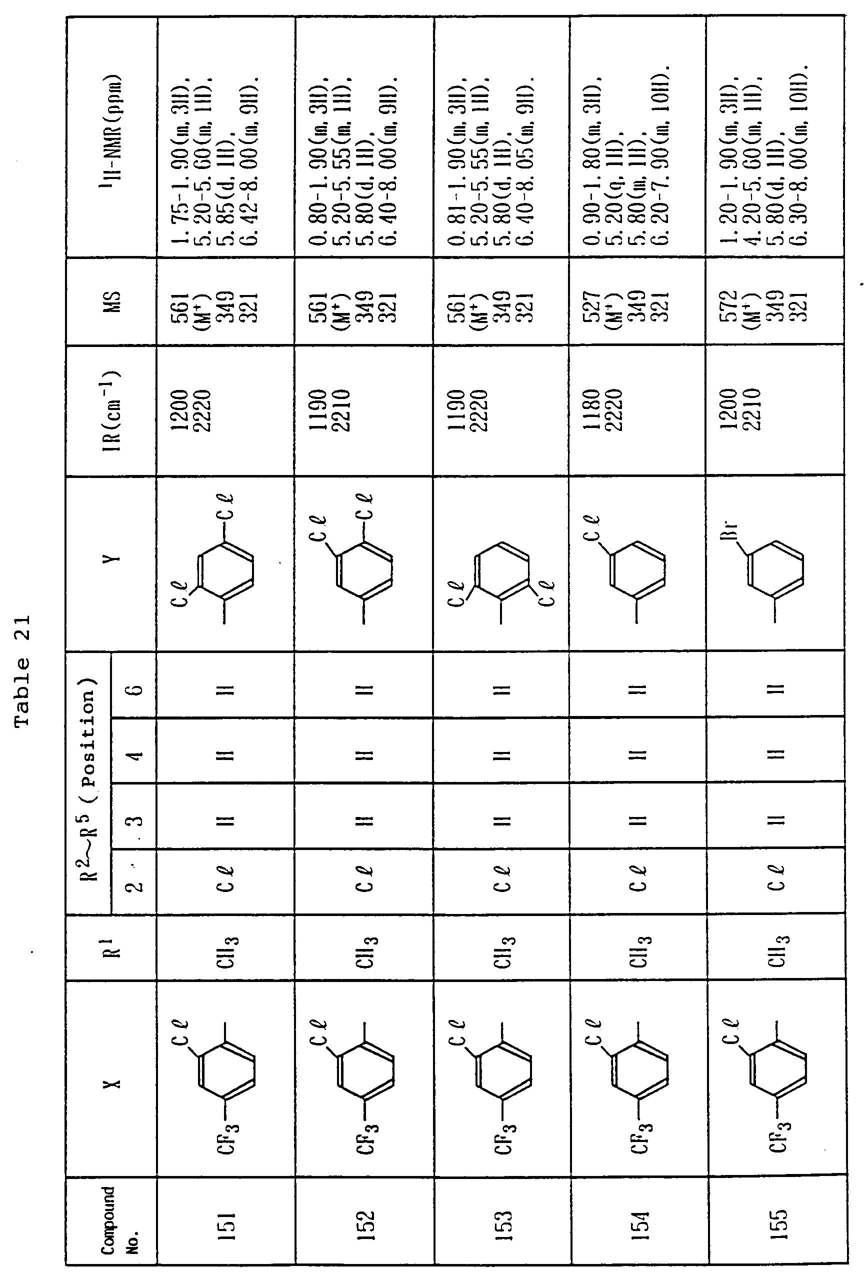

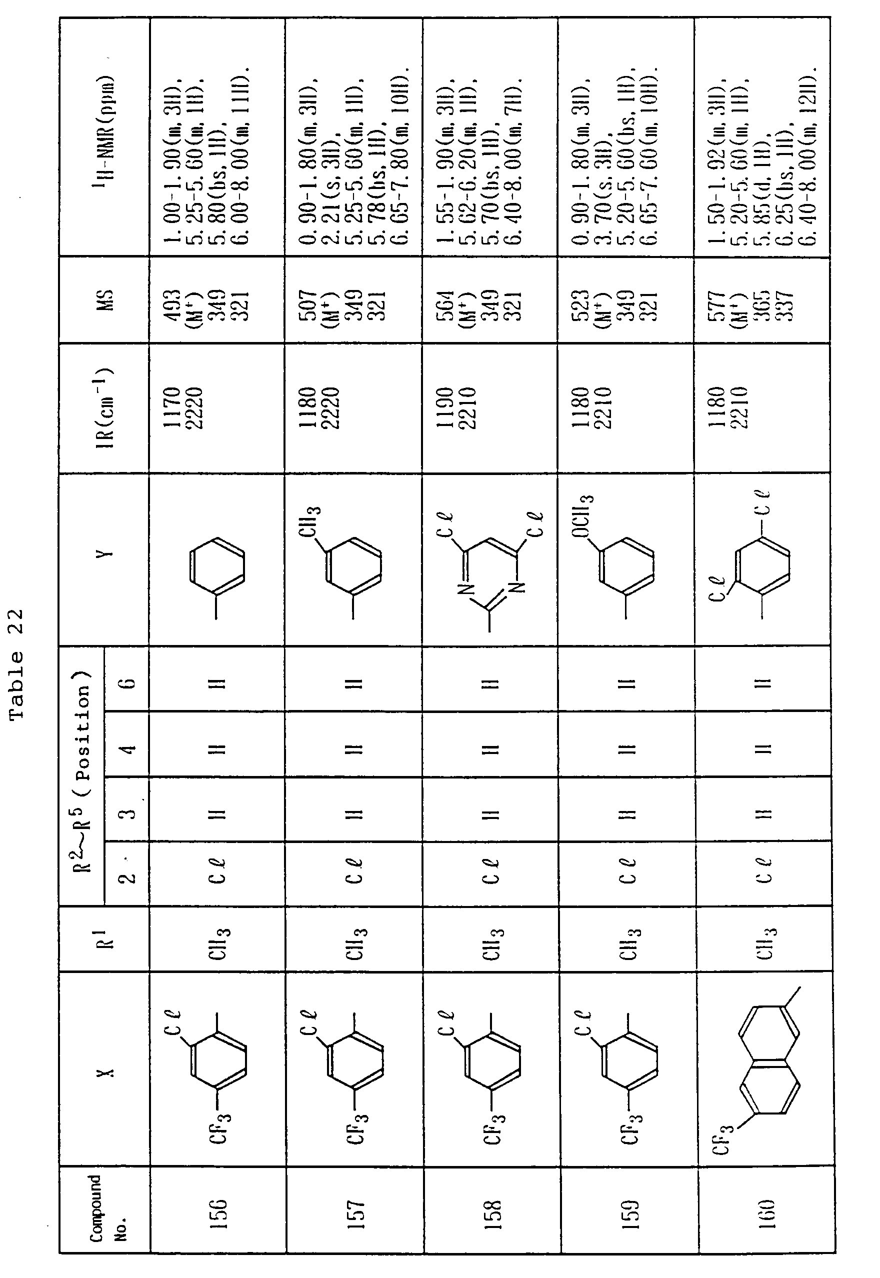

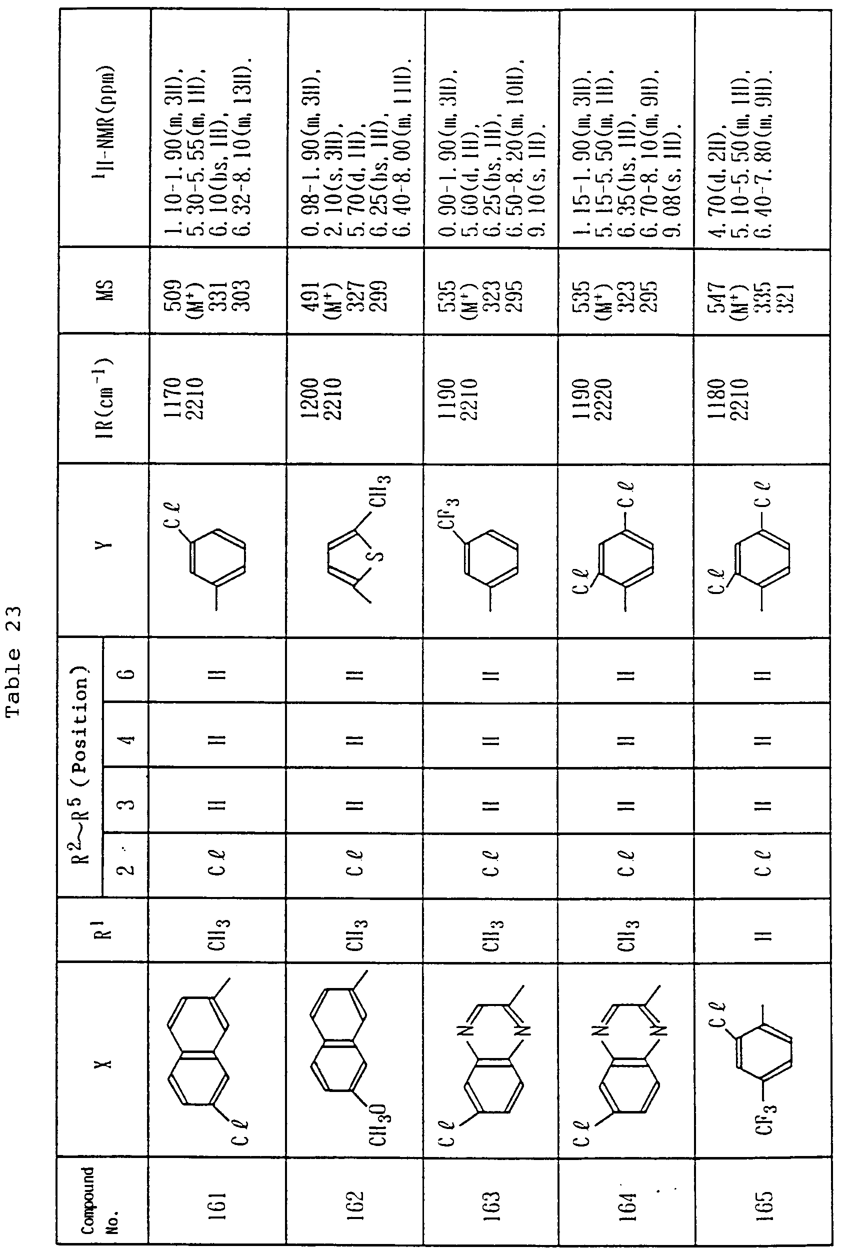

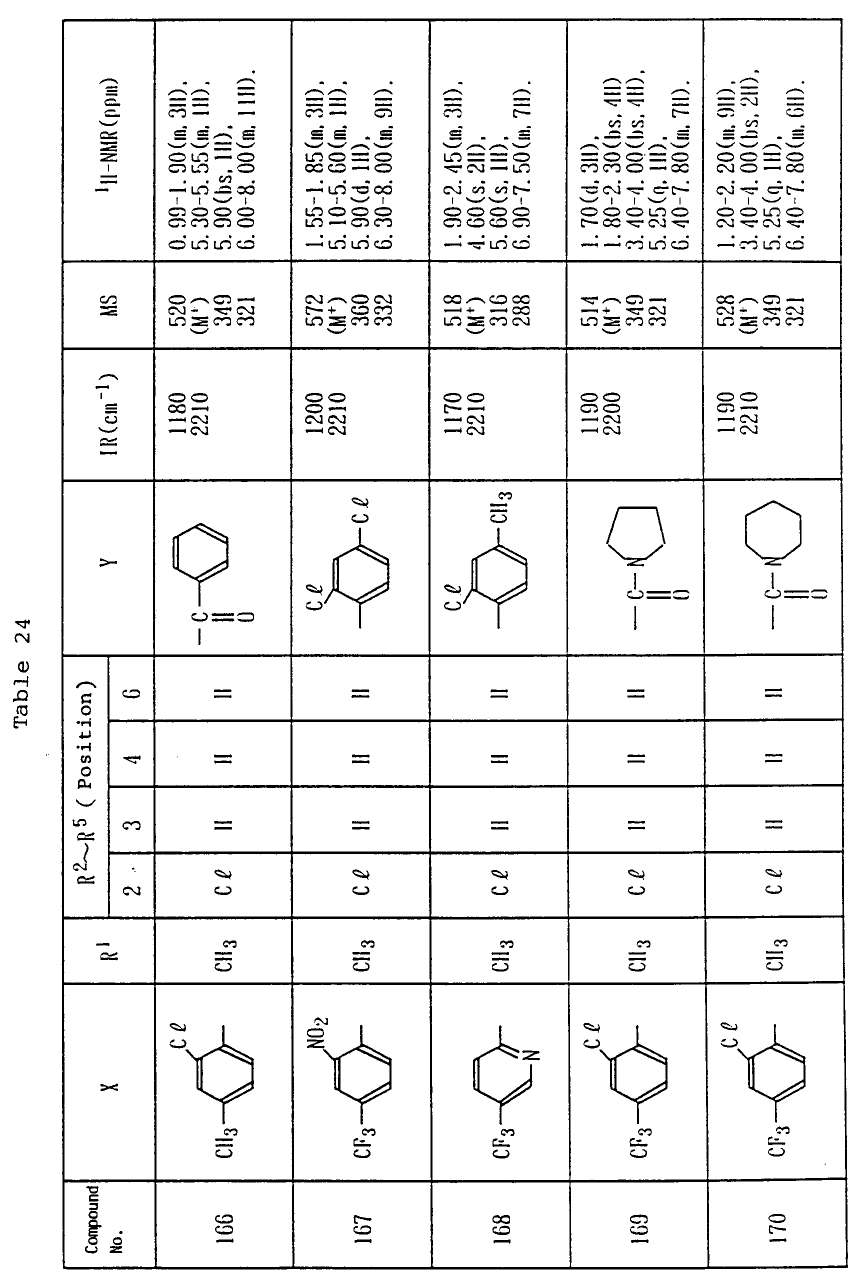

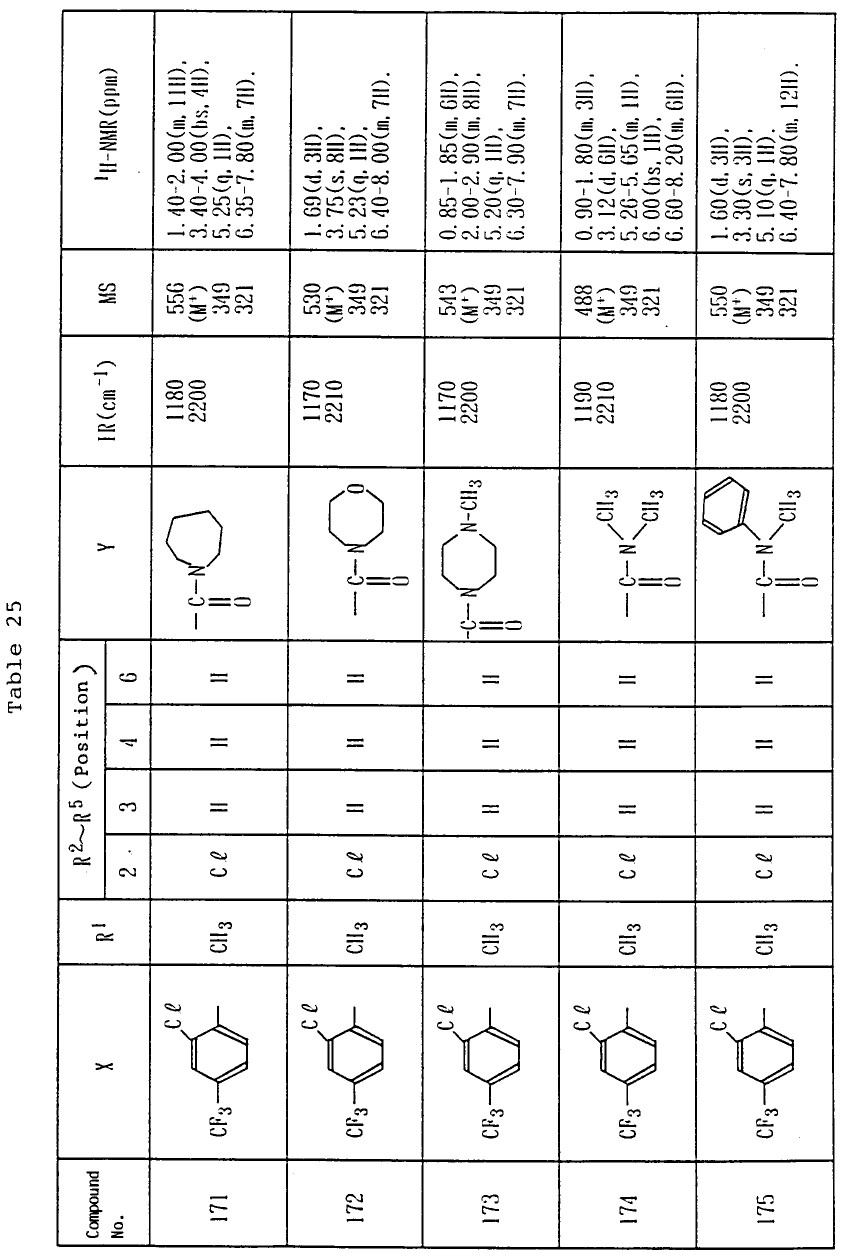

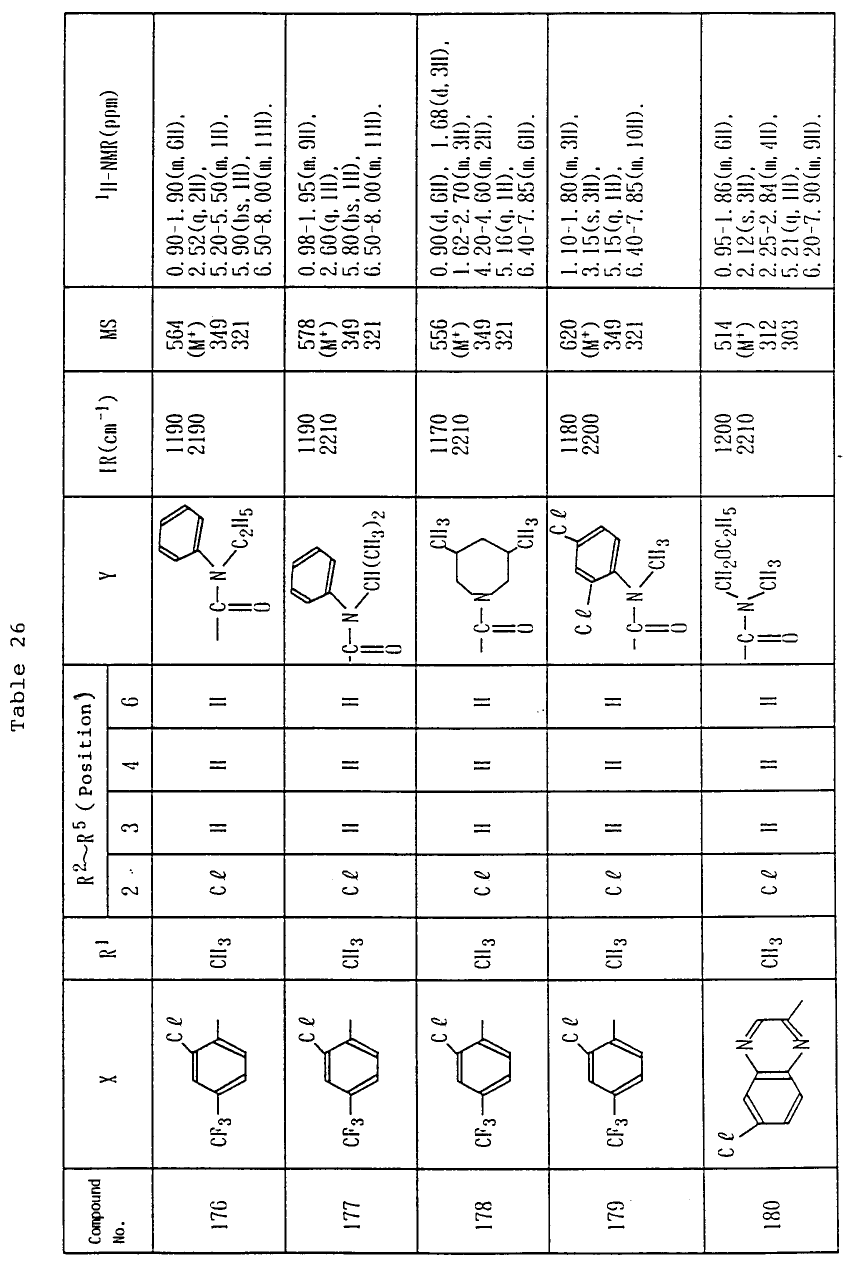

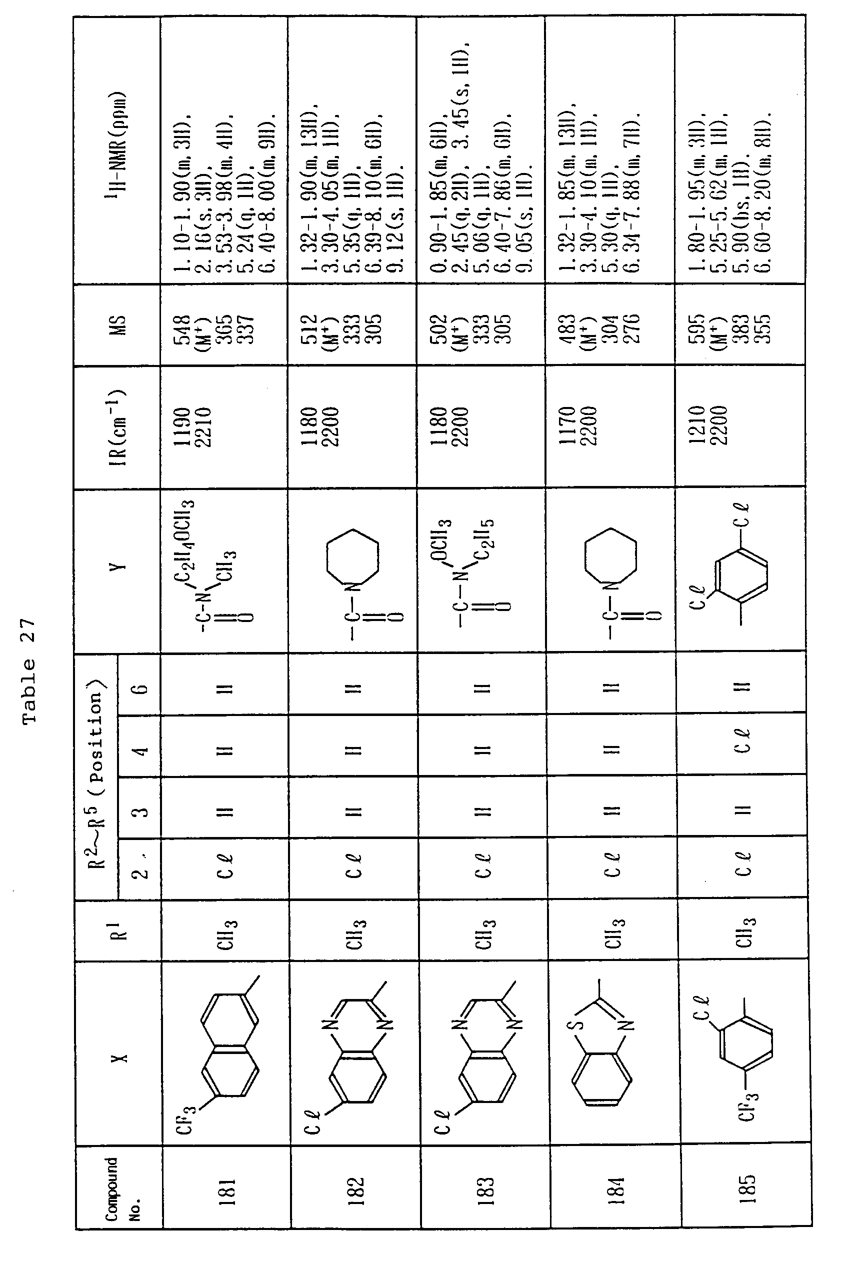

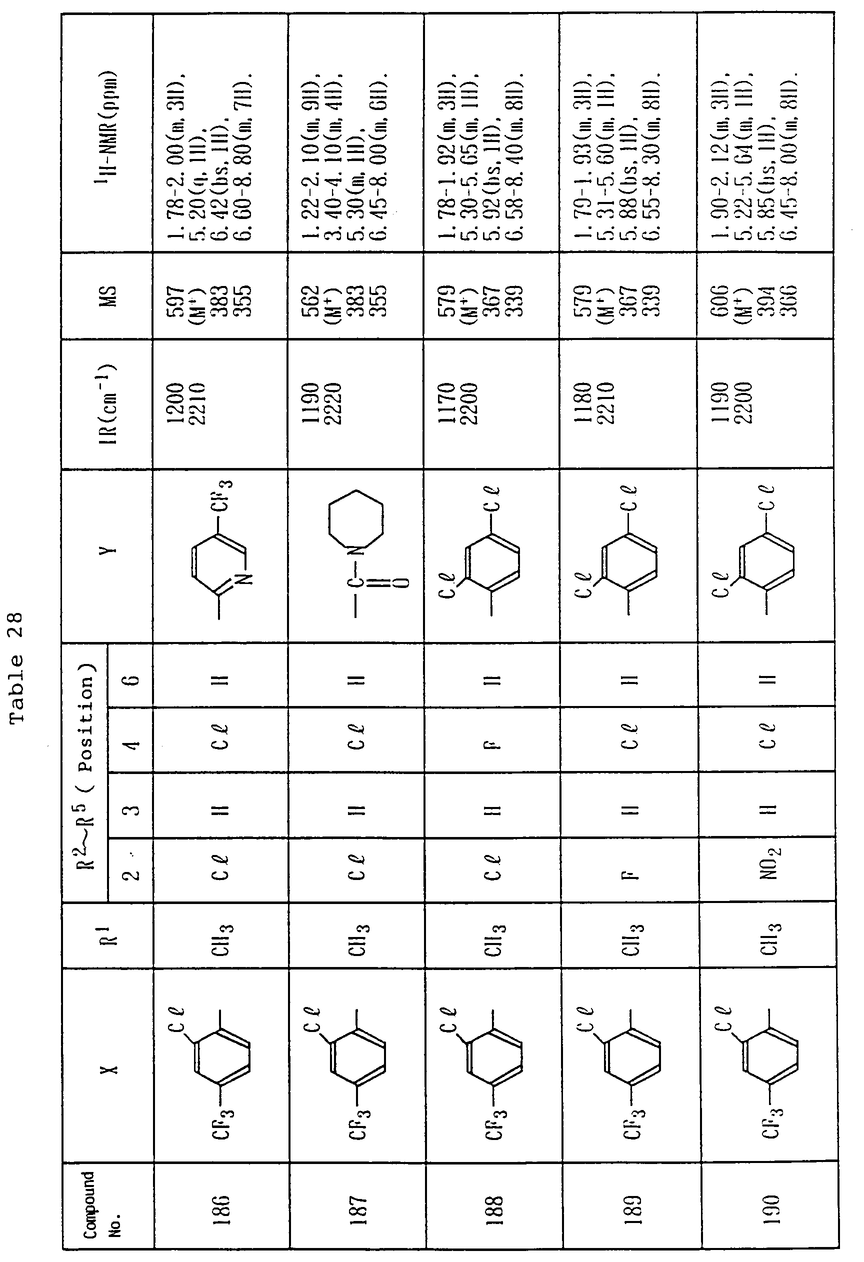

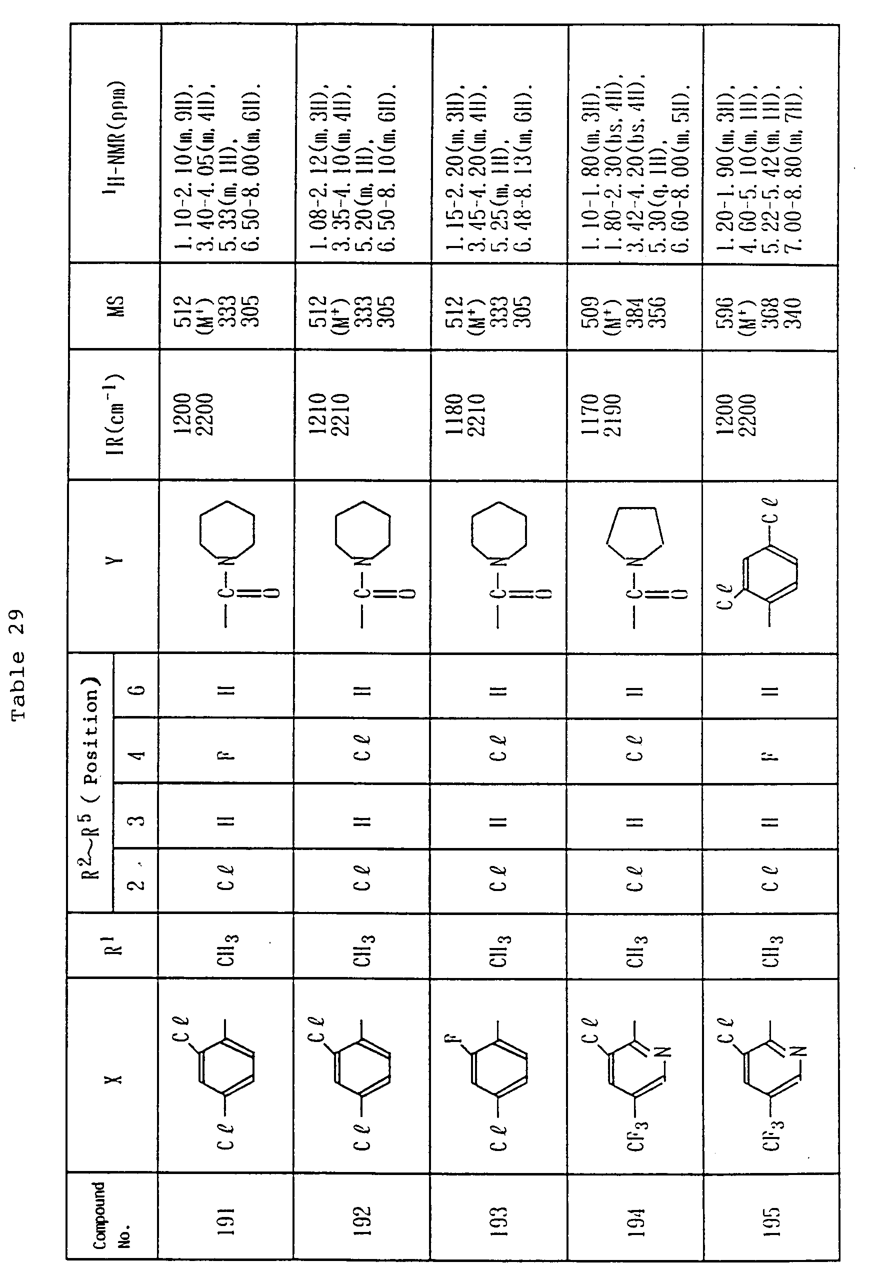

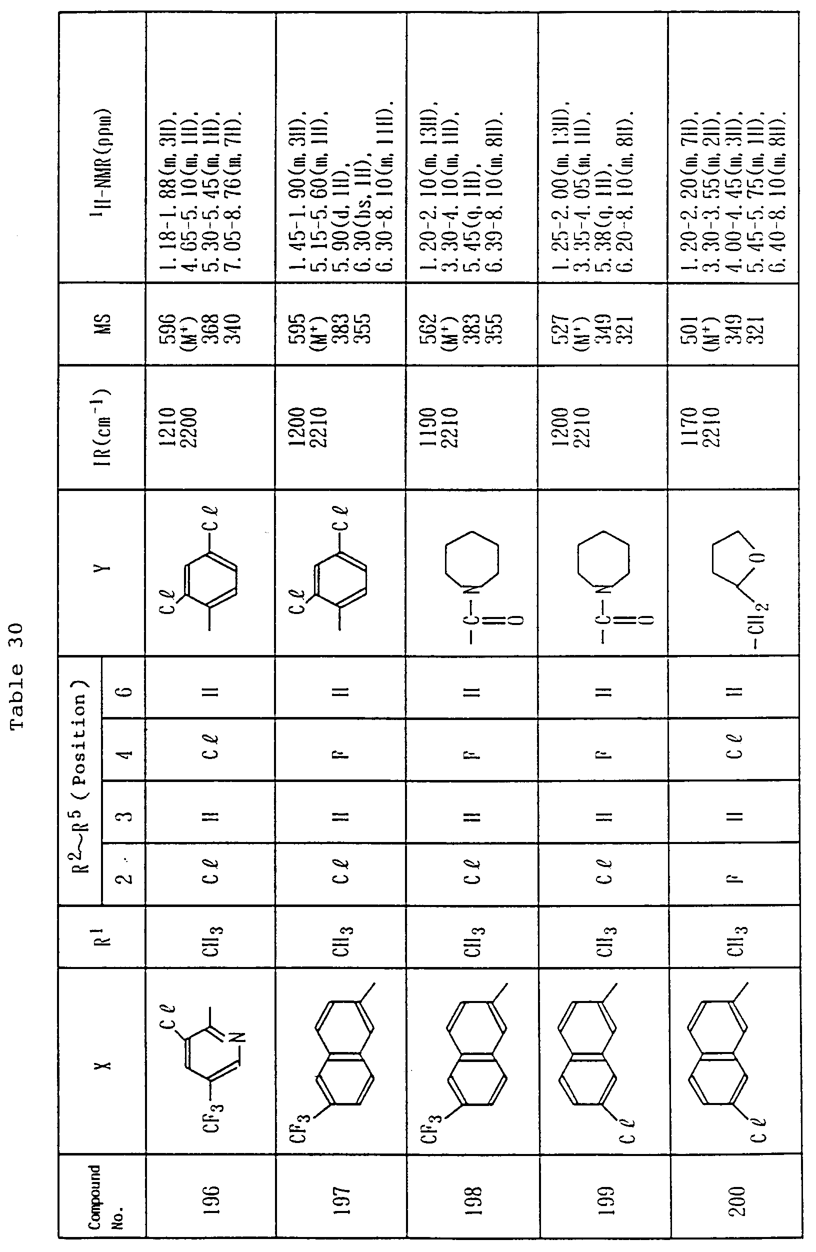

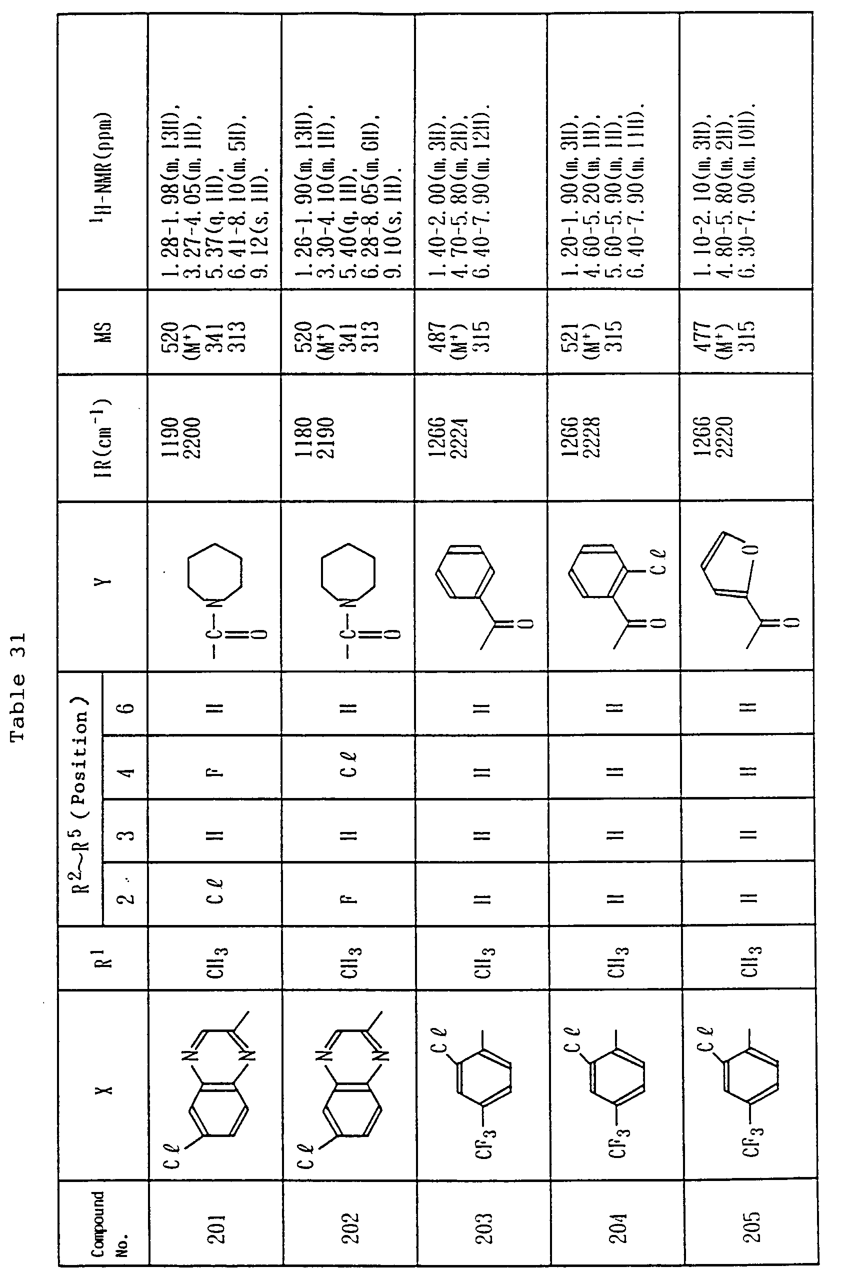

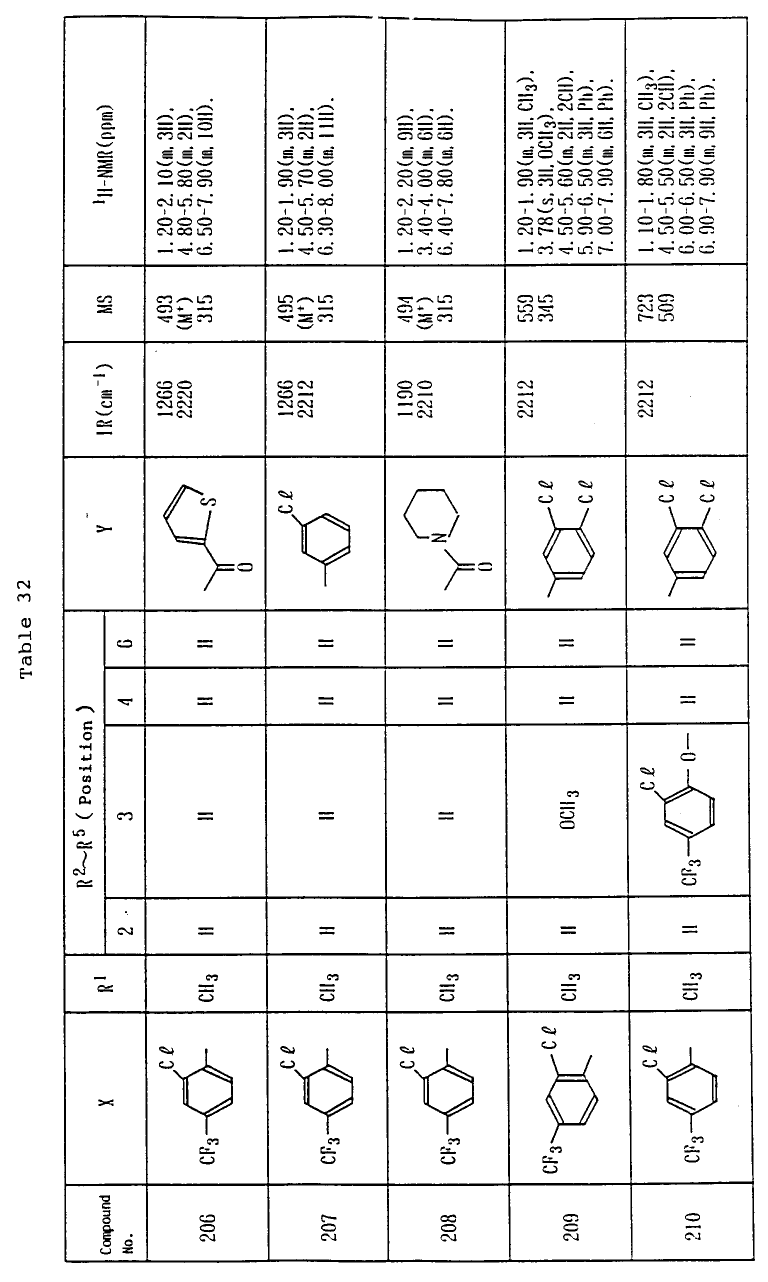

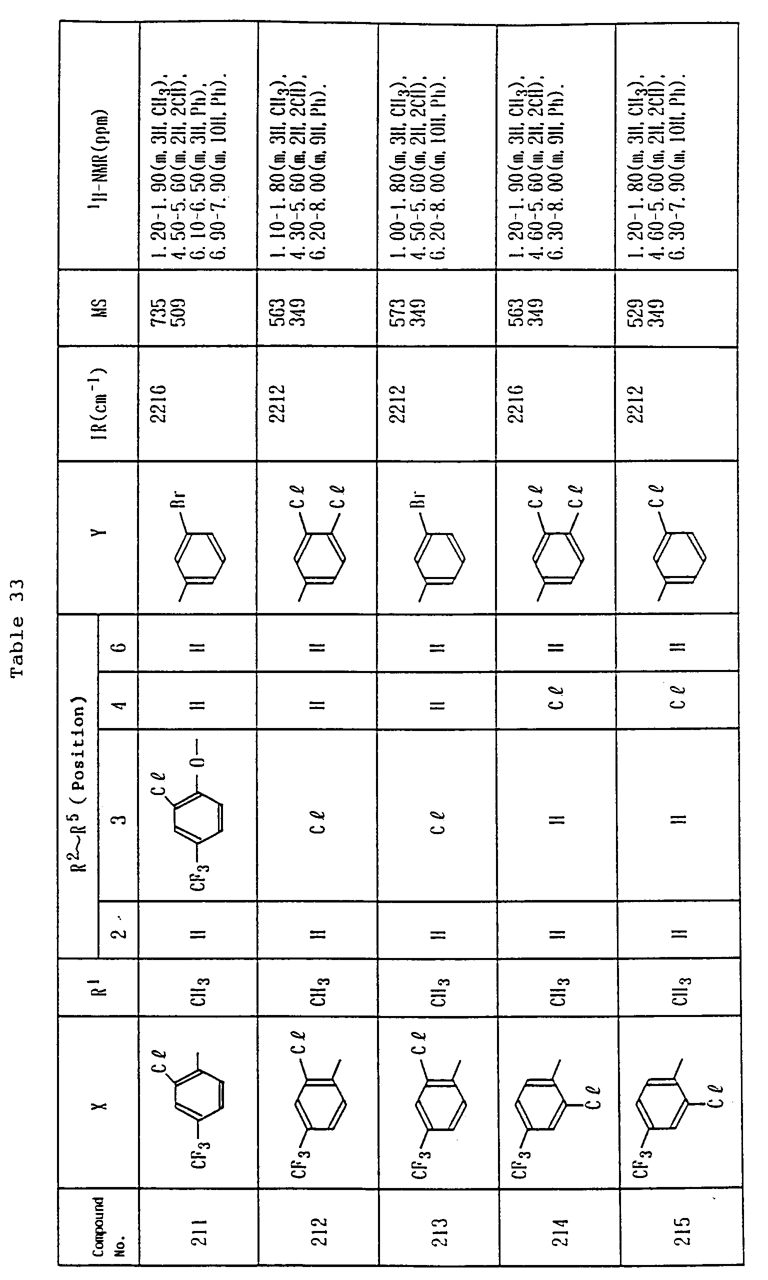

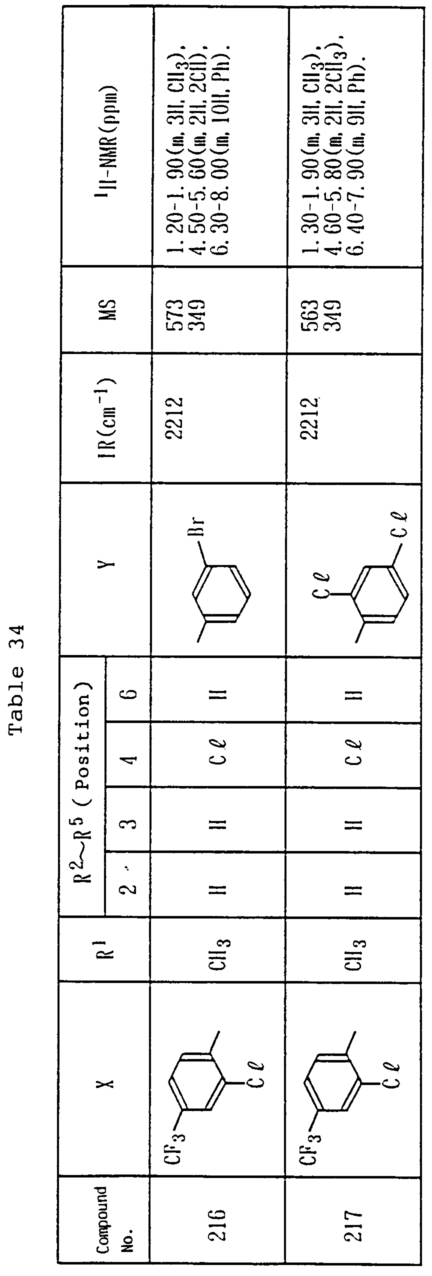

### Preparation Example 25:

4-Methylcatechol (12.4 g) was dissolved in hexamethyl phosphoric acid amide. To the resulting mixture, sodium hydride (oily matter, 70%) (3.4 g), copper powder (3.0 g), and 3,4-dichlorobenzotrifluoride (21.5 g) were added, and stirred at 160°C for 4 hours. The copper powder was removed from the solution by filtration, and ether was added to the filtrate. The ether layer was washed with water, after which ether was distilled off under reduced pressure. The residual crude product was purified by silica gel column chromatography, to afford 2.6 g of a mixture of 2-(2-chloro-4-trifluoromethylphenoxy)-4-methylphenol and 2-(2-chloro-4-trifluoromethylphenoxy)-5-methylphenol. The mixture was dissolved in dimethylformamide. To the solution, sodium hydride (0.34 g) and methyl 2-chloropropionate (1.1 g) were added and stirred at 80°C for 2 hours. After the mixture was cooled, ether was added, and the ether layer was washed with water. The ether was distilled off under reduced pressure, and the resulting crude product was purified by silica gel column chromatography to give 2.2 g of a mixture of methyl 2-(2-(2-chloro-4-trifluoromethylphenoxy)-4-methylphenoxy)propionate and methyl 2-[2-(2-chloro-4-trifluoromethylphenoxy)-5-methylphenoxy]propionate. This mixture was dissolved in ethanol, and combined with 1.1 g of 3,4-dichlorobenzyl cyanide. Sodium ethoxide prepared from metal sodium (0.4 g) was added and heated under reflux for 3 hours. After the reaction mixture was cooled, ether was added. The ether layer was washed with water, and the ether was distilled off under reduced pressure. The resultant crude product was purified by silica gel column chromatography. As a result, 0.5 g of a mixture of 4-[2-(2-chloro-4-trifluoromethylphenoxy)-4-methylphenoxy]-2-(3,4-dichlorophenyl)-3-oxopentanonitrile and 4-[2-(2-chloro-4-trifluoromethylphenoxy)-5-methylphenoxy]-2-(3,4-dichlorophenyl)-3-oxopentanonitrile was obtained (Compound No. 308).

### Preparation Example 26:

In a manner similar to that described in Preparation Example 25, compound Nos. 301 to 310 were prepared.

The chemical structure of Compound Nos. 301 to 310 including the compound obtained in Preparation Example 25 is represented by the following formula.

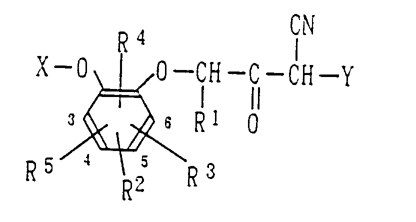

The analytical data obtained with respect to these compounds are shown in Tables 35 and 36, in which substituents R¹, R², R³, R⁴, R⁵, X, and Y are also tabulated. In the Tables, the IR spectral data are characteristic absorption values based on ether bond and cyano group, and the mass spectral data are the molecular ion peaks (M⁺) and fragments. The numbers indicating the positions of substitution of R² through R⁵ in Tables 35 and 36 are shown in the benzene ring of the above formula, and as will be noted, these numbers do not correspond to the numbers in the nomenclature of compounds.

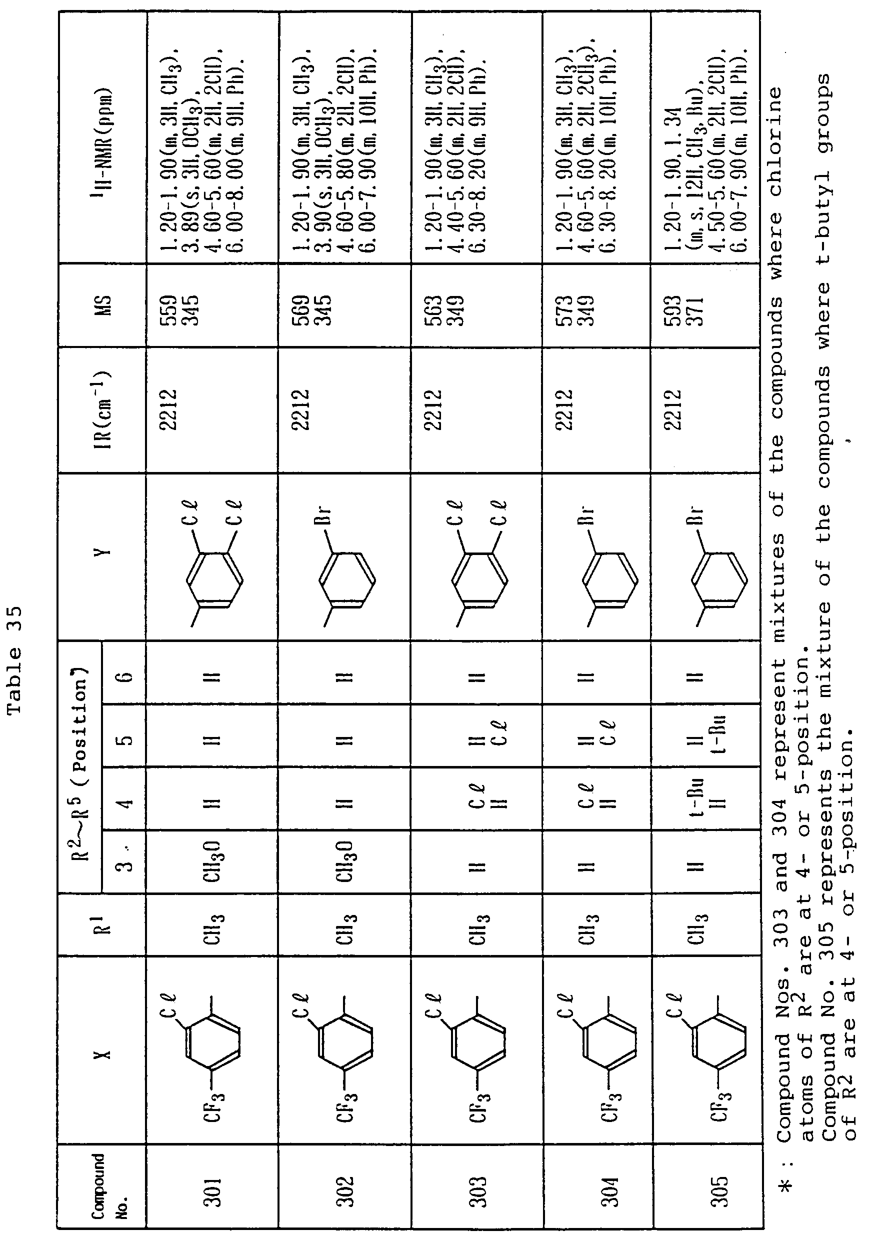

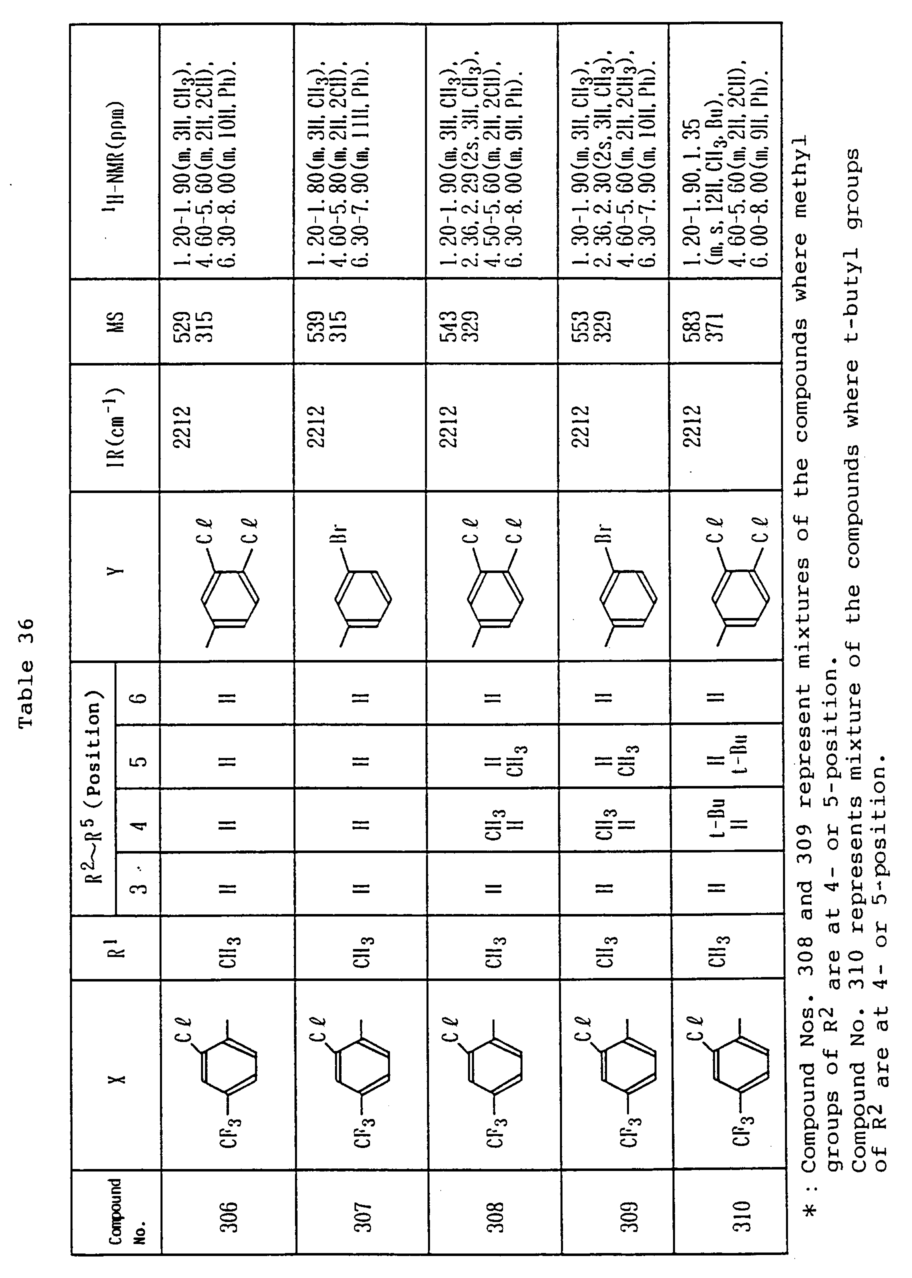

### Example 1 (Capsules for humans):

The following ingredients were charged in capsules to prepare a capsule preparation.

**Table 37**

| (Ingredients) | (Amount, mg) |
|---|---|
| Ketonitrile derivative (Compound No. 4) | 100 |
| Corn starch | 23 |
| CMC-Ca | 22.5 |
| Hydroxymethylcellulose | 3 |
| Magnesium stearate | 1.5 |
| Total | 150.0 |

### Example 2 (Powdery additive for feed):

The following ingredients were mixed to prepare a powdery additive for feed.

**Table 38**

| (Ingredients) | (Amount, g) |
|---|---|
| Ketonitrile derivative (Compound No. 4) | 5-20 |
| Light silicic acid anhydride | 0.5 |
| Corn starch | 94.5-79.5 |
| Total | 100.0 |

### Example 3 (Acute toxicity test):

Acute toxicity (LD₅₀) of a ketonitrile derivative of the present invention (Compound No. 4) was tested using male mice (Slc:ddY, 5 weeks old). To groups of each 5 mice, the compounds suspended in aqueous 0.5% CMC solution were forcibly dosed orally at a single time so that the final dosages of 707, 1,000, 1,414, and 2,000 mg/kg were attained. The mice were observed for 14 days. As a result, LD₅₀ value under conditions of oral administration was estimated to be higher than 2,000 mg/kg. Thus, the compound was confirmed to be quite non-toxic.

### Example 4:

A suitable amount of a sample was dissolved in dimethylsulfoxide (DMSO), and the resultant solution was diluted with the same amount of sterilized distilled water to prepare a bulk sample of 1,000 µg/ml. Mueller-Hinton broth (product of DIFCO) was dispensed into each well of 96-well microplate. Thereafter, the bulk specimen was added to the first well of a row, and successively diluted (10-fold each time) with sterilized distilled water. Separately, a bacterial cell suspension for the test (*Staphylococcus aureus* 209-P) which had been cultured in the above-mentioned broth overnight at 37°C was inoculated to each well so that the final inoculation amount of 10⁶ CFU/ml was attained. The final concentrations of the samples were 100, 10, and 1 µg/ml.

The antibacterial action was evaluated by the inhibitory concentration (hereinafter abbreviated as IC) at which propagation of the bacteria cultured at 37°C for 18 hours was inhibited. Inhibition was determined by the turbidity of the medium. The expressions "IC≤100", "≤10", and "≤1" indicate that MIC-equivalent values obtained at the succesive 2-fold dilution of the sample are 100-10 µg/ml, 10-1 µg/ml, and not more than 1 µg/ml, respectively. The results are shown in Table 39. In Table 39, the expression "≤100", "≤10", and "≤1" indicates that the compound inhibits propagation of the bacteria at a concentration of the compound of not more than 100 µg/ml, 10 µg/ml, and 1 µg/ml, respectively. (In the subsequent Examples, the same rule applies correspondingly.)

**Table 39**

| Antibacterial activity against *Staphylococcus aureus* 209-P | | | | |
|---|---|---|---|---|
| Sample No. ( IC ) | Sample No. ( IC ) | Sample No. ( IC ) | Sample No. ( IC ) | Sample No. ( IC ) |
| 1 (≤10) | 12 (≤100) | 22 (≤10) | 33 (≤10) | 44 (≤1) |
| 2 (≤100) | 13 (≤100) | 23 (≤100) | 34 (≤10) | 45 (≤10) |
| 3 (≤10) | 14 (≤100) | 24 (≤100) | 35 (≤10) | 46 (≤10) |
| 4 (≤1) | 15 (≤10) | 25 (≤100) | 37 (≤100) | 47 (≤10) |
| 5 (≤10) | 16 (≤100) | 26 (≤100) | 38 (≤10) | 308 (≤1) |
| 6 (≤100) | 17 (≤100) | 27 (≤10) | 39 (≤100) | 309 (≤10) |
| 7 (≤100) | 18 (≤100) | 29 (≤100) | 40 (≤100) | |
| 8 (≤100) | 19 (≤100) | 30 (≤100) | 41 (≤1) | |
| 10 (≤10) | 20 (≤100) | 31 (≤100) | 42 (≤1) | |
| 11 (≤100) | 21 (≤100) | 32 (≤100) | 43 (≤10) | |

As is apparent from Table 39, all the tested samples effectively inhibited growth of the bacteria at concentrations not more than 100 µg/ml. In addition, considerable number of samples inhibited growth of the bacteria at concentrations not more than 10 µg/ml or not more than 1 µg/ml.

### Example 5:

In a manner similar to that described in Example 4, antibacterial activity of the invention compound was determined. The bacterium used for the test was *Staphylococcus aureus* TI-1. The results are shown in Table 40.

**Table 40**

| Antibacterial activity against *Staphylococcus aureus* TI-1 | | | | |
|---|---|---|---|---|
| Sample No. ( IC ) | Sample No. ( IC ) | Sample No. ( IC ) | Sample No. ( IC ) | Sample No. ( IC ) |
| 1 (≤10) | 12 (≤10) | 22 (≤10) | 33 (≤10) | 46 (≤1) |
| 2 (≤10) | 13 (≤100) | 24 (≤100) | 34 (≤10) | 47 (≤1) |
| 3 (≤10) | 14 (≤100) | 25 (≤100) | 35 (≤100) | 308 (≤1) |
| 4 (≤1) | 15 (≤10) | 26 (≤100) | 36 (≤10) | 309 (≤1) |
| 5 (≤10) | 16 (≤10) | 27 (≤10) | 37 (≤100) | |
| 6 (≤100) | 17 (≤100) | 28 (≤100) | 41 (≤1) | |
| 7 (≤10) | 18 (≤10) | 29 (≤100) | 42 (≤1) | |
| 8 (≤10) | 19 (≤100) | 30 (≤100) | 43 (≤10) | |
| 10 (≤10) | 20 (≤10) | 31 (≤100) | 44 (≤1) | |
| 11 (≤100) | 21 (≤10) | 32 (≤100) | 45 (≤1) | |

As is apparent from Table 40, all the tested samples effectively inhibited growth of the bacteria at concentrations not more than 100 µg/ml. In addition, considerable number of samples inhibited growth of the bacteria at concentrations not more than 10 µg/ml or 1 µg/ml.

### Example 6:

In a manner similar to that described in Example 4, antibacterial activity of the invention compound was determined. The bacterium used for the test was *Pasteurella Kobe*-6 (origin: pig; *Pasteurella multocida*). The results are shown in Table 41.

**Table 41**

| Antibacterial activity against *Pasteurella multocid*a (Kobe-6) | | | |
|---|---|---|---|
| Sample No. ( IC ) | Sample No. ( IC ) | Sample No. ( IC ) | Sample No. ( IC ) |
| 1 (≤100) | 15 (≤100) | 41 (≤10) | 47 (≤100) |
| 2 (≤100) | 16 (≤100) | 42 (≤10) | 308 (≤10) |
| 3 (≤100) | 17 (≤100) | 43 (≤10) | 309 (≤10) |
| 4 (≤100) | 20 (≤100) | 44 (≤10) | |
| 5 (≤100) | 22 (≤100) | 45 (≤10) | |
| 10 (≤100) | 27 (≤100) | 46 (≤100) | |

As is apparent from Table 41, all the tested samples effectively inhibited growth of the gram-negative mactirium, *Pasteurella multocida* at concentrations not more than 100 µg/ml.

### Example 7:

In a manner similar to that described in Example 4, antibacterial activity of the invention compound was determined. The bacterium used for the test was *Bordetella bronchiseptica* (SM2-4). The results are shown in Table 42.

**Table 42**

| Antibacterial activity against *Bordetella bronchiseptica* (SM2-4) | | | |
|---|---|---|---|
| Sample No. ( IC ) | Sample No. ( IC ) | Sample No. ( IC ) | Sample No. ( IC ) |
| 2 (≤100) | 9 (≤100) | 14 (≤100) | 16 (≤100) |
| 41 (≤100) | 42 (≤100) | 43 (≤100) | 44 (≤100) |
| 45 (≤100) | 46 (≤100) | 47 (≤100) | 308 (≤100) |

As is apparent from Table 42 all the tested samples effectively inhibited growth of the gram-negative bacterium *Bordetella bronchiseptica* at concentrations not more than 100 µg/ml.

### Example 8:

A suitable amount of a sample was dissolved in dimethylsulfoxide (DMSO), after which the resulting solution was diluted with the same amount of sterilized distilled water to prepare a bulk sample of 1,000 µg/ml. Separately, a bacterial liquid for the test (methicillin-resistant *Staphylococcus aureus* (MRSA) isolated from humans) which had been cultured in Mueller-Hinton broth (product of DIFCO) was prepared and inoculated to a Mueller-Hinton agar (product of DIFCO) containing the sample so that the final inoculation amount of 10⁶ CFU/ml was achieved. The final concentrations of the samples were 100, 10, and 1 µg/ml.

After culturing at 37°C for 18 hours, the antibacterial activity was evaluated in terms of IC in a manner similar to that described in Example 4. The results are shown in Table 43.

**Table 43**

| Antibacterial activity of various compounds against methicillin-resistan*t Staphylococcus aureus* (MRSA) isolated from humans | | | | | | | | | |
|---|---|---|---|---|---|---|---|---|---|
| Strains | Compound numbers | | | | | | | | |
| | 41 | 42 | 43 | 44 | 45 | 46 | 47 | 308 | 309 |
| 900056 | ≤1 | ≤10 | ≤1 | ≤1 | ≤10 | ≤1 | ≤10 | ≤1 | ≤1 |
| 900048 | ≤1 | ≤1 | ≤1 | ≤1 | ≤10 | ≤1 | ≤10 | ≤1 | ≤1 |
| 891187 | ≤1 | ≤1 | ≤1 | ≤1 | ≤10 | ≤1 | ≤10 | ≤1 | ≤1 |
| 891185 | ≤1 | ≤1 | ≤1 | ≤1 | ≤10 | ≤10 | ≤10 | ≤1 | ≤1 |
| 891192 | ≤1 | ≤1 | ≤10 | ≤1 | ≤10 | ≤10 | ≤10 | ≤1 | ≤1 |
| 891179 | ≤1 | ≤1 | ≤1 | ≤1 | ≤10 | ≤10 | ≤10 | ≤1 | ≤10 |

As is apparent from Table 43, all the tested samples exhibited an inhibitory activity against MRSA at concentrations not more than 10 µg/ml.

### Example 9:

Through use of a ketonitrile derivative of the present invention (Compound No. 4) as a sample, minimum inhibitory concentrations (MIC) of *Staphylococcus aureus* and *Pasteurella* from cattle were determined by an agar plate dilution method in accordance with the standard procedure authorized by Japan Chemotherapy Association ["Chemotherapy", 29, 76-79, 1981, under the heading "Revised Measurement of Minimum Inhibitory Concentration (MIC)"]. The samples were dissolved in dimethylsulfoxide and diluted with the same amount of sterilized distilled water to prepare a bulk sample liquid of 1,000 µg/ml. The bulk was diluted into a series of 2-fold dilutions using sterilized distilled water.

Separately, the bacterial cell suspensions for the test shown in Table 18 were cultured in Mueller-Hinton broth (DIFCO) at 37°C for 18-20 hours in advance. They were controlled to have a bacterial amount of 10⁶ CFU/ml by use of Mueller-Hinton broth. The thus-controlled liquids were inoculated to a Muller-Hinton agar plate containing the sample, followed by culturing at 37°C for 18 hours. The concentration at which bacterial growth was no longer observed was taken as the MIC. The results are shown in Table 44.

**Table 44**

| Antibacterial activity of Compound No. 4 against *Staphylococcus aureus* and *Pasteurella* from cattle | | |
|---|---|---|
| Tested bacteria | Origin | MIC (µg/ml) |
| Staphylococcus aureus 209P | Standard strain | 1.56 |
| Staphylococcus aureus TI-1 | Cattle | 6.25 |
| Staphylococcus aureus 3-2 | Pig | 3.13 |
| Staphylococcus aureus 1SS1 | Chicken | 3.13 |
| Pasteurella haemolytica 1F-15 | Cattle | 6.25 |
| Pasteurella Multosida 1F-17 | Cattle | 1.56 |

As is apparent from Table 44, MIC values are in the range from 1.56 to 6.25 µg/ml, revealing high antibacterial activity against respective bacteria tested.

### Example 10:

Through use of a ketonitrile derivative of the present invention (Compound No. 4) as a sample, minimum inhibitory concentrations (MIC) of respective bacterial strains were determined by a microliquid dilution method in accordance with the standard procedure authorized by Japan Chemotherapy Association ["Chemotherapy", 29, 76-79, 1981, under the heading "Revised Measurement of Minimum Inhibitory Concentration (MIC)"]. The samples were dissolved in dimethylsulfoxide and diluted with the same amount of sterilized distilled water to prepare a bulk sample liquid of 1,000 µg/ml. The bulk sample was cultured in Mueller-Hinton broth at 37°C for 18 hours. The results are shown in Table 45.

**Table 45**

| Antibacterial activity of Compound No. 4 against various bacterial strains | |
|---|---|
| Tested bacteria | MIC (µg/ml) |
| Staphylococcus aureus SMITH | 3.13 |
| * Staphylococcus aureus M5-1 | 3.13 |
| * Staphylococcus aureus M5-2 | 3.13 |
| Staphylococcus epidermis 56556 | 6.25 |
| Streptococcus pyogenes G-36 | 6.25 |
| Streptococcus mitis 11D 685 | 12.5 |
| Enterococcus faecalis ATCC 19433 | 6.25 |
| Bacillus subtilis ATCC 6633 | 3.13 |

| | |
|---|---|
| *: Methicillin-resistant *Staphylococcus aureus* | |

As is apparent from Table 45, the present derivative exhibited high activities against pathological gram-positive bacteria including methicillin-resistant *Staphylococci*. Especially, the present derivative was effective against methicillin-resistant *Staphylococcus aureus*.

### Example 11 (Capsules for humans):

The following ingredients were charged in capsules to prepare a capsule preparation.

**Table 46**

| (Ingredients) | (Amount, mg) |
|---|---|
| Ketonitrile derivative (Compound No. 155) | 100 |
| Corn starch | 23 |
| CMC-Ca | 22.5 |
| Hydroxymethylcellulose | 3 |
| Magnesium stearate | 1.5 |
| Total | 150.0 |

### Example 12 (Powdery additive for feed):

The following ingredients were mixed to prepare a powdery additive for feed.

**Table 47**

| (Ingredients) | (Amount, g) |
|---|---|
| Ketonitrile derivative (Compound No. 155) | 5-20 |
| Light silicic acid anhydride | 0.5 |
| Corn starch | 94.5-79.5 |
| Total | 100.0 |

### Example 13 (Acute toxicity test):

Acute toxicity (LD₅₀) of ketonitrile derivatives of the present invention (Compound Nos. 103 and 155) was tested using male mice (Slc:ddY, 5 weeks old). To groups of each 5 mice, the compounds suspended in aqueous 0.5% CMC solution were forcibly dosed orally at a single time so that the final dosages of 500, 1,000, 1,500, and 2,000 mg/kg were attained. The mice were observed for 14 days. As a result, the LD₅₀ value was estimated to be higher than 2,000 mg/kg. Thus, the compounds were confirmed to be quite non-toxic.

### Example 14 (Absorption test):

An absorption test was performed using ketonitrile derivatives of the present invention (Compound Nos. 103 and 155) on mice by dosing the derivatives in amounts of 100 mg/kg body weight. One, three, five, and 24 hours after administration of the compounds, blood was harvested and serum was separated therefrom. The concentration of the compounds in serum was determined by HPLC. As a result, Compound No. 155 and Compound No. 101 reached a maximum in concentration after 1 hour and 3 hours of administration, respectively. Thus, both ketonitrile derivatives were found to be quickly absorbed by oral administration.

### Example 15:

In a manner similar to that described in Example 8, antibacterial activity of the ketonitrile derivatives shown in Table 48 was determined. The bacterium used for the test was *Staphylococcus aureus* (209-P). The results are shown in Table 48.

**Table 48**

| Antibacterial activity against *Staphylococcus aureus* 209-P | | | | |
|---|---|---|---|---|
| Sample No. ( IC ) | Sample No. ( IC ) | Sample No. ( IC ) | Sample No. ( IC ) | Sample No. ( IC ) |
| 102 (≤10) | 109 (≤10) | 137 (≤100) | 171 (≤10) | 211 (≤10) |
| 103 (≤1) | 110 (≤10) | 151 (≤1) | 172 (≤100) | 212 (≤1) |
| 104 (≤1) | 111 (≤10) | 152 (≤1) | 175 (≤10) | 213 (≤10) |
| 105 (≤10) | 112 (≤1) | 155 (≤1) | 207 (≤1) | 214 (≤10) |
| 106 (≤10) | 116 (≤10) | 156 (≤10) | 208 (≤10) | 215 (≤10) |
| 107 (≤100) | 134 (≤100) | 169 (≤100) | 209 (≤10) | 216 (≤10) |
| 108 (≤10) | 136 (≤100) | 170 (≤10) | 210 (≤10) | 217 (≤10) |

As is apparent from Table 48, all the tested samples effectively inhibited growth of the bacteria at concentrations not more than 100 µg/ml. In addition, considerable number of samples inhibited growth of the bacteria at concentrations not more than 10 µg/ml or 1 µg/ml.

### Example 16:

In a manner similar to that described in Example 8, antibacterial activity of the invention compound was determined. The bacterium used for the test was *Streptococcus aureus* (TI-1). The results are shown in Table 49.

**Table 49**

| Antibacterial activity against *Streptococcus aureus* (TI-1) | | | | |
|---|---|---|---|---|
| Sample No. ( IC ) | Sample No. ( IC ) | Sample No. ( IC ) | Sample No. ( IC ) | Sample No. ( IC ) |
| 102 (≤1) | 108 (≤10) | 134 (≤100) | 170 (≤10) | 212 (≤1) |
| 103 (≤1) | 109 (≤1) | 137 (≤100) | 207 (≤1) | 213 (≤10) |
| 104 (≤1) | 110 (≤10) | 151 (≤1) | 208 (≤10) | 214 (≤1) |
| 105 (≤10) | 111 (≤10) | 152 (≤1) | 209 (≤10) | 215 (≤1) |
| 106 (≤10) | 112 (≤1) | 155 (≤1) | 210 (≤10) | 216 (≤1) |
| 107 (≤100) | 116 (≤10) | 169 (≤100) | 211 (≤10) | 217 (≤10) |
| 108 (≤10) | 136 (≤100) | 170 (≤10) | 210 (≤10) | 217 (≤10) |

As is apparent from Table 49, all the tested samples effectively inhibited growth of the bacteria at concentrations not more than 100 µg/ml. In addition, considerable number of samples inhibited growth of the bacteria at concentrations not more than 10 µg/ml or 1 µg/ml.

### Example 17:

In a manner similar to that described in Example 8, antibacterial activity of the invention compound was determined. The bacterium used for the test was *Pasteurella Kobe*-6 (origin: pig, *Pasteurella multocida*). The results are shown in Table 50.

**Table 50**

| Antibacterial activity against *Pasteurella multocida* (Kobe-6) | | | | |
|---|---|---|---|---|
| Sample No. ( IC ) | Sample No. ( IC ) | Sample No. ( IC ) | Sample No. ( IC ) | Sample No. ( IC ) |
| 103 (≤10) | 109 (≤10) | 134 (≤100) | 155 (≤100) | 211 (≤10) |
| 104 (≤10) | 110 (≤100) | 135 (≤100) | 156 (≤100) | 212 (≤10) |
| 105 (≤100) | 111 (≤100) | 137 (≤100) | 170 (≤100) | 213 (≤10) |
| 106 (≤100) | 112 (≤1) | 151 (≤100) | 209 (≤10) | 214 (≤100) |
| 108 (≤100) | 116 (≤10) | 152 (≤10) | 210 (≤100) | 215 (≤10) |
| - | - | - | - | 216 (≤100) |
| - | - | - | - | 217 (≤100) |

As is apparent from Table 50, all the tested samples effectively inhibited growth of the gram-negative bacterium *Pasteurella multocida* at concentrations not more than 100 µg/ml.

### Example 18:

In a manner similar to that described in Example 8, antibacterial activity of the invention compound was determined. The bacterium used for the test was *Bordetella bronchiseptica* (S1) and *Bordetella bronchiseptica* (SM2-4). The results are shown in Table 51.

**Table 51**

| Antibacterial activity against *Bordetella bronchiseptica* (S1) | |
|---|---|
| Sample No. ( IC ) | Sample No. ( IC ) |
| 112 (≤100) | 212 (≤100) |
| 155 (≤100) | 213 (≤100) |

| Antibacterial activity against *Bordetella bronchiseptica* (SM1-4) | |
|---|---|
| Sample No. ( IC ) | Sample No. ( IC ) |
| 112 (≤100) | 212 (≤100) |
| 155 (≤100) | 213 (≤100) |

As is apparent from Table 51, the tested samples effectively inhibited growth of the gram-negative bacterium *Bordetella bronchiseptica* at concentrations not more than 100 µg/ml.

### Example 19:

In a manner similar to that described in Example 8, antibacterial activity of a variety of ketonitrile derivatives shown in Table 52 was measured. The bacterium used for the test was methicillin-resistant *Staphylococcus aureus* (MRSA) from humans. The results are shown in Table 52.

**Table 52**

| Antibacterial activity of various compounds against methicillin-resistant *Staphylococcus aureus* (MRSA) isolated from humans | | | | | | | | | |
|---|---|---|---|---|---|---|---|---|---|
| Strain/sample | Compound number | | | | | | | | |
| | 103 | 104 | 105 | 109 | 110 | 111 | 112 | 155 | |
| 900056 | ≤ 1 | ≤10 | ≤10 | ≤10 | ≤10 | ≤10 | ≤ 1 | ≤ 1 | |
| 900048 | ≤ 1 | ≤10 | ≤10 | ≤10 | ≤10 | ≤10 | ≤ 1 | ≤ 1 | |
| 891187 | ≤ 1 | ≤10 | ≤10 | ≤10 | ≤10 | ≤10 | ≤ 1 | ≤ 1 | |
| 891185 | ≤ 1 | ≤10 | ≤10 | ≤10 | ≤10 | ≤10 | ≤ 1 | ≤ 1 | |
| 891192 | ≤ 1 | ≤10 | ≤10 | ≤10 | ≤10 | ≤10 | ≤ 1 | ≤ 1 | |
| 891179 | ≤ 1 | ≤10 | ≤10 | ≤10 | ≤10 | ≤10 | ≤ 1 | ≤ 1 | |

| Strain/sample | Compound number | | | | | | | | |
|---|---|---|---|---|---|---|---|---|---|
| | 209 | 210 | 211 | 212 | 213 | 214 | 215 | 216 | 217 |
| 900056 | ≤10 | ≤10 | ≤10 | ≤ 1 | ≤10 | ≤ 1 | ≤ 1 | ≤ 1 | ≤10 |
| 900048 | ≤10 | ≤10 | ≤10 | ≤ 1 | ≤10 | ≤ 1 | ≤10 | ≤ 1 | ≤10 |
| 891187 | ≤10 | ≤10 | ≤10 | ≤ 1 | ≤ 1 | ≤ 1 | ≤10 | ≤ 1 | ≤10 |
| 891185 | ≤10 | ≤10 | ≤10 | ≤ 1 | ≤ 1 | ≤ 1 | ≤ 1 | ≤ 1 | ≤10 |
| 891192 | ≤10 | ≤10 | ≤10 | ≤ 1 | ≤ 1 | ≤10 | ≤ 1 | ≤10 | ≤10 |
| 891179 | ≤10 | ≤10 | ≤10 | ≤ 1 | ≤10 | ≤ 1 | ≤10 | ≤10 | ≤10 |

As is apparent from Table 52, all the tested samples effectively inhibited bacterial growth of MRSA at concentrations not more than 10 µg/ml.

### Example 20:

In a manner similar to that described in Example 8, antibacterial activity of a variety of ketonitrile derivatives shown in Tables 53-55 was measured. The bacteria used for the test were *Enterococcus* and *Pasteurella* from fish. The culture conditions were such that the media contained 1.5% NaCl, and the culture temperature was 25°C. The results are shown in Tables 53-55.

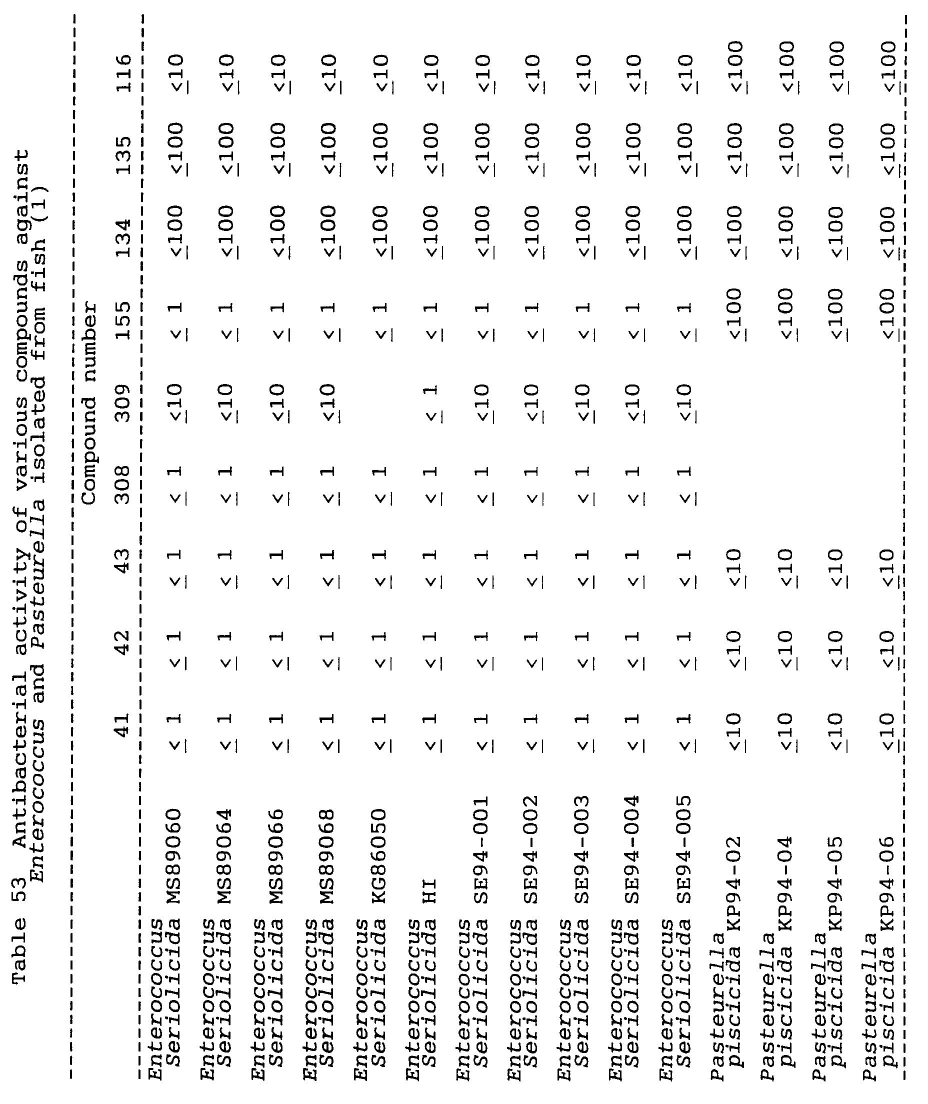

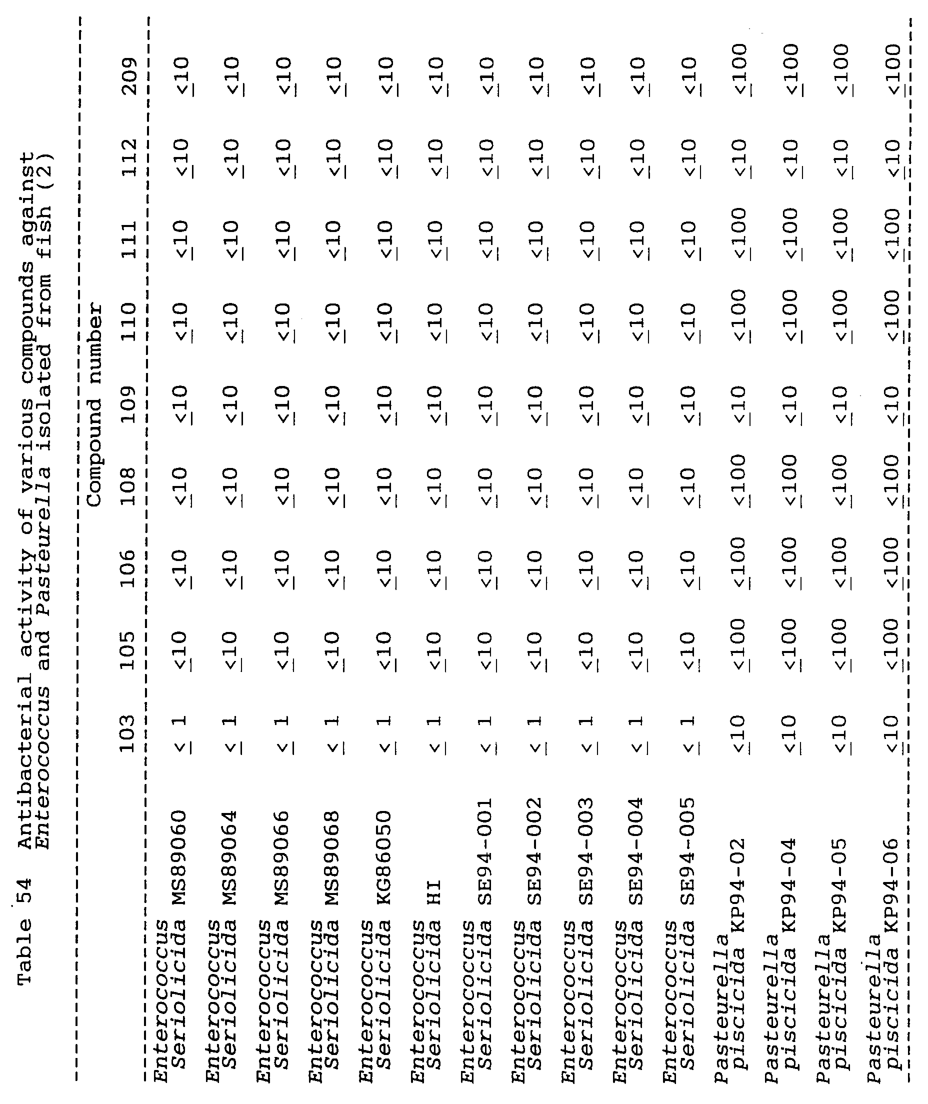

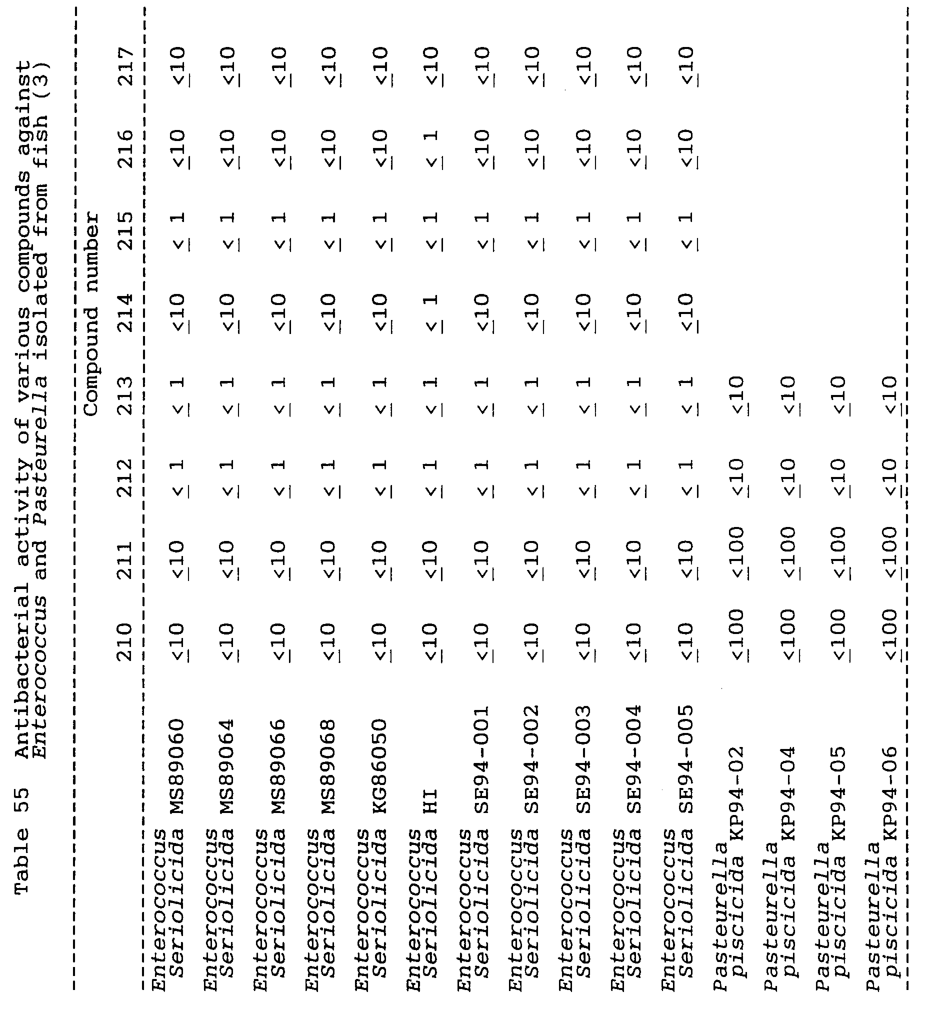

As is apparent from Tables 53-55, all the tested samples effectively inhibited bacterial growth of various bacteria at concentrations not more than 100 µg/ml.

### Industrial Applicability

As described hereinbefore, the antibacterial agent of the present invention possesses excellent antibacterial activity against gram-positive and gram-negative bacteria. It is especially noteworthy that the antibacterial agent of the invention is effective against pathological *Staphylococci* including methicillin-resistant *Staphylococcus aureus* (MRSA). Therefore, the agent of the invention can be used in the prevention and therapeutical treatment of various infectious diseases of humans and animals including mammals except humans, poultry, and fish caused by gram-positive bacteria or gram-negative bacteria.

## Claims

1. An antibacterial agent containing, as an active component, a ketonitrile derivative represented by the following formula (1) or a salt thereof: 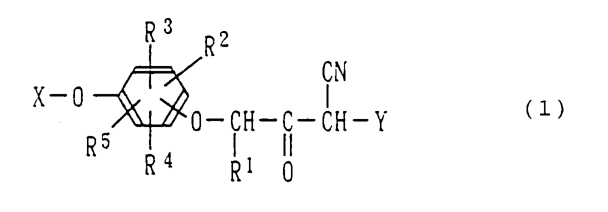 (wherein R¹ is a hydrogen atom or lower alkyl, each of R², R³, R⁴, and R⁵, which are identical to or different from one another, is a hydrogen atom, a halogen atom, nitro, lower alkyl, lower alkoxyl, or substituted or unsubstituted phenoxy, X is a substituted or unsubstituted aromatic hydrocarbon group or a substituted or unsubstituted heterocyclic group, Y is a substituted or unsubstituted aromatic hydrocarbon group, a substituted or unsubstituted heterocyclic group, a carbonyl group substituted by a substituted or unsubstituted aromatic hydrocarbon group or by a substituted or unsubstituted heterocyclic group, or a N,N-di-substituted carbamoyl group.

2. An antibacterial agent containing, as an active component, a ketonitrile derivative represented by the following formula (2) or a salt thereof: 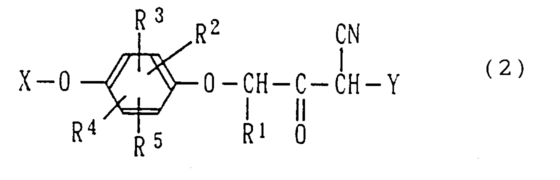 (wherein R¹ is a hydrogen atom or lower alkyl, each of R², R³, R⁴, and R⁵, which are identical to or different from one another, is a hydrogen atom, a halogen atom, nitro, lower alkyl, lower alkoxyl, or substituted or unsubstituted phenoxy, X is a substituted or unsubstituted aromatic hydrocarbon group or a substituted or unsubstituted heterocyclic group, Y is a substituted or unsubstituted aromatic hydrocarbon group, a substituted or unsubstituted heterocyclic group, a carbonyl group substituted by a substituted or unsubstituted aromatic hydrocarbon group or by a substituted or unsubstituted heterocyclic group, or a N,N-di-substituted carbamoyl group.

3. An antibacterial agent containing, as an active component, a ketonitrile derivative represented by the following formula (3) or a salt thereof: 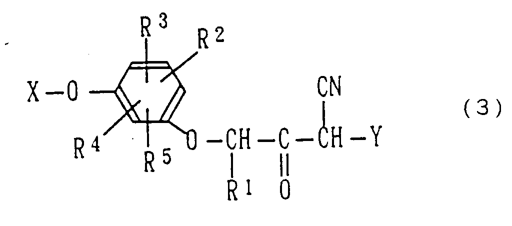 (wherein R¹ is a hydrogen atom or lower alkyl, each of R², R³, R⁴, and R⁵, which are identical to or different from one another, is a hydrogen atom, a halogen atom, nitro, lower alkyl, lower alkoxyl, or substituted or unsubstituted phenoxy, X is a substituted or unsubstituted aromatic hydrocarbon group or a substituted or unsubstituted heterocyclic group, Y is a substituted or unsubstituted aromatic hydrocarbon group, a substituted or unsubstituted heterocyclic group, a carbonyl group substituted by a substituted or unsubstituted aromatic hydrocarbon group or by a substituted or unsubstituted heterocyclic group, or a N,N-di-substituted carbamoyl group.

4. An antibacterial agent containing, as an active component, a ketonitrile derivative represented by the following formula (4) or a salt thereof: 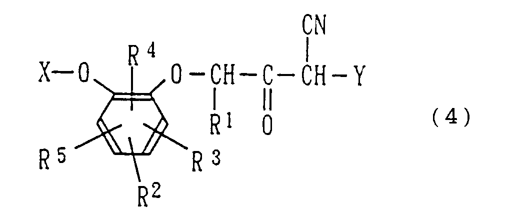 (wherein R¹ is a hydrogen atom or lower alkyl, each of R², R³, R⁴, and R⁵, which are identical to or different from one another, is a hydrogen atom, a halogen atom, nitro, lower alkyl, lower alkoxyl, or substituted or unsubstituted phenoxy, X is a substituted or unsubstituted aromatic hydrocarbon group or a substituted or unsubstituted heterocyclic group, Y is a substituted or unsubstituted aromatic hydrocarbon group, a substituted or unsubstituted heterocyclic group, a carbonyl group substituted by a substituted or unsubstituted aromatic hydrocarbon group or by a substituted or unsubstituted heterocyclic group, or a N,N-di-substituted carbamoyl group.

5. The antibacterial agent according to any one of Claims 1 through 4, wherein each of X and Y is substituted or unsubstituted aromatic hydrocarbon group or substituted or unsubstituted aromatic heterocyclic group.

6. The antibacterial agent according to any one of Claims 1 through 4, wherein X is substituted or unsubstituted phenyl, substituted or unsubstituted pyridyl, substituted or unsubstituted benzoxazolyl, or substituted or unsubstituted benzothiazolyl, Y is substituted or unsubstituted phenyl, substituted or unsubstituted pyrimidinyl, substituted or unsubstituted thienyl, or substituted or unsubstituted naphthyl, or N,N-di-substituted carbamoyl.

7. The antibacterial agent according to any one of Claims 1 through 4, wherein X is phenyl which may be substituted by a halogen atom, haloalkyl, or cyano; pyridyl which may be substituted by a halogen atom, haloalkyl, or cyano; benzoxazolyl which may be substituted by a halogen atom, haloalkyl, or cyano; or benzothiazolyl which may be substituted by a halogen atom, haloalkyl, or cyano; Y is phenyl which may be substituted by a halogen atom, haloalkyl, alkyl, or alkoxyl; pyrimidinyl which may be substituted by a halogen atom, haloalkyl, alkyl, or alkoxyl; thienyl which may be substituted by a halogen atom, haloalkyl, alkyl, or alkoxyl; or naphthyl which may be substituted by a halogen atom, haloalkyl, alkyl, or alkoxyl; N,N-di-substituted carbamoyl that has been substituted by lower alkyl, aryl, or aralkyl; or 1-azacycloalkylcarbonyl which may be condensed with a benzene ring or which may be substituted by an alkyl group.

8. The antibacterial agent according to Claim 2, wherein, in formula (2), X is substituted or unsubstituted pyridyl, and Y is substituted or unsubstituted phenyl; substituted or unsubstituted pyrimidinyl, or N,N-di-substituted carbamoyl.

9. The antibacterial agent according to Claim 2, wherein, in formula (2), X is substituted or unsubstituted phenyl and Y is substituted or unsubstituted pyrimidinyl or N,N-di-substituted carbamoyl.

10. The antibacterial agent according to Claim 2, wherein, in formula (2), X is substituted or unsubstituted benzoxazolyl or substituted or unsubstituted benzothiazolyl and Y is substituted or unsubstituted phenyl.

11. An antibacterial agent containing, as an active component, 4-[4-(3-chloro-5-trifluoromethyl-2-pyridyloxy)phenoxy]-2-(3,4-dichlorophenyl)-3-oxopentanonitrile, 2-(3-bromophenyl)-4-[2-chloro-5-(2-chloro-4-trifluoromethylphenoxy)phenoxy]-3-oxopentanonitrile, 4-[3-(2-chloro-4-trifluoromethylphenoxy)phenoxy]-2-(3,4-dichlorophenyl)-3-oxopentanonitrile, 4-[3-(2-chloro-4-trifluoromethylphenoxy)-5-methylphenoxy]-2-(3,4-dichlorophenyl)-3-oxopentanonitrile, 4-[2-(2-chloro-4-trifluoromethylphenoxy)-4-methylphenoxy]-2-(3,4-dichlorophenyl)-3-oxopentanonitrile, 4-[2-(2-chloro-4-trifluoromethylphenoxy)-5-methylphenoxy]-2-(3,4-dichlorophenyl)-3-oxopentanonitrile, 4-[2-(2-chloro-4-trifluoromethylphenoxy)-4-methylphenoxy]-2-(3-bromophenyl)-3-oxopentanonitrile, 4-[2-(2-chloro-4-trifluoromethylphenoxy)-5-methylphenoxy]-2-(3-bromophenyl)-3-oxopentanonitrile, or a salt of any one of these compounds.

12. The antibacterial agent according to Claim 3, wherein, in formula (3), X is a substituted or unsubstituted aromatic hydrocarbon group or a substituted or unsubstituted aromatic heterocyclic group.

13. The antibacterial agent according to Claim 3, wherein, in formula (3), X is substituted or unsubstituted phenyl or substituted or unsubstituted pyridyl.

14. The antibacterial agent according to Claim 3, wherein, in formula (3), X is phenyl which has been substituted by a halogen atom or haloalkyl; or pyridyl which has been substituted by a halogen atom or haloalkyl.

15. The antibacterial agent according to Claim 3, wherein, in formula (3), Y is substituted or unsubstituted phenyl, substituted or unsubstituted naphthyl, substituted or unsubstituted pyridyl, or N,N-di-substituted carbamoyl.

16. The antibacterial agent according to Claim 3, wherein, in formula (3), Y is phenyl which has been substituted by a halogen atom or haloalkyl.

17. The antibacterial agent according to Claim 3, wherein, in formula (3), X is substituted phenyl or substituted pyridyl, Y is substituted or unsubstituted phenyl, substituted or unsubstituted pyridyl, or N,N-di-substituted carbamoyl.

18. The antibacterial agent according to Claim 3, wherein, in formula (3), X is phenyl which has been substituted by a halogen atom or haloalkyl, or pyridyl which has been substituted by halogen or haloalkyl, Y is phenyl which has been substituted by a halogen atom, haloalkyl, or alkoxyl.

19. The antibacterial agent according to Claim 4, wherein, in formula (4), X is substituted or unsubstituted aromatic hydrocarbon or substituted or unsubstituted aromatic heterocyclic.

20. The antibacterial agent according to Claim 4, wherein, in formula (4), X is substituted or unsubstituted phenyl or substituted or unsubstituted pyridyl.

21. The antibacterial agent according to Claim 4, wherein, in formula (4), X is phenyl which has been substituted by a halogen atom or haloalkyl; or pyridyl which has been substituted by a halogen atom or haloalkyl.

22. The antibacterial agent according to Claim 4, wherein, in formula (4), X is phenyl which may be substituted by a halogen atom or haloalkyl.

23. The antibacterial agent according to Claim 4, wherein, in formula (4), Y is substituted or unsubstituted phenyl, substituted or unsubstituted naphthyl, or substituted or unsubstituted pyridyl.

24. The antibacterial agent according to Claim 4, wherein, in formula (4), Y is phenyl which has been substituted by a halogen atom or haloalkyl.

25. A pharmaceutical composition containing a ketonitrile derivative as described in Claim 1 or a salt thereof as well as a pharmaceutically acceptable carrier.

26. An antibacterial pharmaceutical composition containing a ketonitrile derivative as described in Claim 1 or a salt thereof as well as a pharmaceutically acceptable carrier.

27. Use, as an antibacterial agent, of a ketonitrile derivative as described in Claim 1 or a salt thereof.

28. The use according to Claim 27, wherein the antibacterial agent is for animals and humans.
